# EUROPEAN PATENT SPECIFICATION

(11) **EP 4 408 260 B1**
(45) Date of publication and mention of the grant of the patent: **17.12.2025**
(21) Application number: 22802410.5
(22) Date of filing: 29.09.2022
(51) Int. Cl.: A61B 5/00, G01N 33/483, G01N 3/08

(54) **NON-DESTRUCTIVE PRESSURE-ASSISTED TISSUE STIFFNESS MEASUREMENT APPARATUS**
ZERSTÖRUNGSFREIE DRUCKUNTERSTÜTZTE GEWEBESTEIFIGKEITSMESSVORRICHTUNG
APPAREIL DE MESURE DE RIGIDITÉ DE TISSUS ASSISTÉ PAR PRESSION NON DESTRUCTIVE

(30) Priority: 29.09.2021 US 202163250123 P
(43) Date of publication of application: 07.08.2024
(73) Proprietor: The Trustees Of The Stevens Institute Of Technology, Hoboken, New Jersey 07030 (US); Vanderbilt University, Nashville, TN 37240 (US); The Trustees of Columbia University in the City of New York, New York, NY 10027 (US); The Board of Trustees of the Leland Stanford Junior University, Stanford, CA 94305-2038 (US)
(72) Inventor: KIM, Jinho, Jersey City, New Jersey 07302 (US); VUNJAK-NOVAKOVIC, Gordana, New York, New York 10025 (US); O'NEILL, John D., New York, New York 10022 (US); PINEZICH, Meghan, New York, New York 10003 (US); GUENTHART, Brandon A., Stanford, California 94305 (US); MIR, Seyed Mohammad, Hoboken, New Jersey 07030 (US); CHEN, Jiawen, Hoboken, New Jersey 07030 (US); BACCHETTA, Matthew, Nashville, Tennessee 37240 (US)
(74) Representative: Mewburn Ellis LLP
(86) International application number: PCT/US2022/077311
(87) International publication number: WO 2023/056395

(56) References cited:
- WO-A1-03/105689
- DE-A1- 19 650 992
- FR-A1- 2 580 400
- US-A1- 2011 071 436
- US-A1- 2015 173 996
- US-A1- 2017 160 175
- CHEN JIAWEN ET AL: "Non-destructive vacuum-assisted measurement of lung elastic modulus", ACTA BIOMATERIALIA, ELSEVIER, AMSTERDAM, NL, vol. 131, 27 June 2021 (2021-06-27), pages 370 - 380, XP086747168, ISSN: 1742-7061, [retrieved on 20210627], DOI: 10.1016/J.ACTBIO.2021.06.037
- PATRICK SCHIAVONE ET AL: "LASTIC: A Light Aspiration Device for in vivo Soft TIssue Characterization", 23 January 2010, BIOMEDICAL SIMULATION, SPRINGER BERLIN HEIDELBERG, BERLIN, HEIDELBERG, PAGE(S) 1 - 10, ISBN: 978-3-642-11614-8, XP019138186

## Description

### FIELD OF THE INVENTION

The present invention relates to the measurement of mechanical properties, and, specifically, to the determination of elastic modulus of soft tissues, organs, and biomaterials without compromising their native structure.

### BACKGROUND OF THE INVENTION

In biological tissues, mechanical stiffness plays a fundamental role in cell and tissue function. Alterations in the stiffness, or elasticity, of tissues can induce pathological interactions that affect cellular activity and tissue function. The integral connection between tissue stiffness and disease highlights the importance of accurate quantitative characterizations of soft tissue mechanics, which can improve our understanding of disease and inform therapeutic development. For example, accurate evaluation of the mechanical properties of lung tissue has been especially challenging due to its anatomical and mechanobiological complexities. Discrepancies in the measured mechanical properties of dissected lung tissue samples and intact lung tissue in vivo have limited the ability to accurately characterize intrinsic lung mechanics.

Current devices and methods for measuring the stiffness of soft tissues are limited to the surface of accessible tissues and require the operator to rely on their vision to place the devices. This limits the ability of researchers and surgeons to understand both healthy and diseased tissue properties, which understanding is critical in advancing diagnostics and treatments of soft tissue diseases.

US2017160175A1 proposes methods and systems for measuring at least one mechanical characteristic of a soft material. Devices and methods for estimating tissue elasticity are proposed in the publication by Schiavone et al entitled "LASTIC: A Light Aspiration Device for in vivo Soft Tissue Characterization", Lecture Notes in Computer Science, vol 5958. Springer, Berlin, Heidelberg. US2011071436A1 proposes a sensor for detecting the force applied to or by a soft material to thereby measure the stiffness of the soft material, for example during surgical procedures. The sensor comprises a sphere that floats on a cushion of air. The displacement of the sphere is measured by optical means. The sensor is integrated in a catheter and the catheter tip may also be heated and used for ablation of tissue.

### SUMMARY OF THE INVENTION

The invention is defined by the appended set of claims.

The present invention involves a device that can measure stiffness of a wide variety of tissues, organs, and biomaterials in a non-destructive and rapid manner, as well as methods of using such a device to quantify tissue or material stiffness. The inventive device is a pressure-assisted device that is able to evaluate the stiffness of biomaterials, tissues, and organs in a non-destructive and/or minimally invasive manner, thereby allowing accurate and rapid quantification of tissue and organ stiffness in vivo and ex vivo. Furthermore, the device can be applied to detect, treat, and/or remove the injured or diseased tissue.

Conventional methods, such as tensile and compression tests, require isolation of tissue samples for the measurements that can result in substantial alteration in native tissue structure and anatomy, leading to inaccurate readouts. The inventive device allows for vacuum or compression-assisted direct in situ measurement of local tissue without the need of tissue sampling, allowing for evaluation of tissues and organs that are difficult to access. The device can be designed with a steerable and conformable configuration such that it can be inserted and placed locally into the measurement sites within the patient's body that are difficult access, such as the respiratory, gastrointestinal, and urinary tracts.

The inventive device is integrated with a miniaturized camera or optical fiber imaging probe that allows clinicians to accurately determine the position of the device during its insertion and navigation within the patient's body, thereby facilitating placement of the device to target locations with improved spatial resolution for stiffness measurements. Furthermore, the measurement device can be conformable, steerable, thin (e.g., diameter less than 5 mm), and long (e.g., length of approximately 1 m), allowing minimally invasive device insertion via a small incision opening created in the patient's body and placement of the device onto any tissue or organ surface for measurements, such as lung, respiratory tract, liver, heart, brain, or intestines.

**In** addition, if the inventive device is configured as a balloon-integrated probe, measurement of internal tissue stiffness can be achieved. When equipped with a balloon, the inventive probe can be introduced locally into the lung tissue via a syringe needle, wherein the balloon can be easily expanded inside the lung tissue by introducing air or fluid. Pressure and volume inside the balloon can be determined in real time via a pressure sensor and a volume sensor, respectively, that are connected to a pump externally, allowing accurate quantification of tissue stiffness.

The present invention contains many possible commercial applications: The inventive probe can alleviate the major challenges encountered during tumor resection surgery that arise due to difficulty identifying the boundaries of the tumor, so that it can be ensured that the entire tumor is removed during surgery. Specifically, the inventive device can serve as an intraoperative tool to determine the margins of a tumor in real-time to facilitate complete removal of tumors. Additionally, there are applications in mechanical testing to evaluate injury and function in donor organs to determine suitability for transplantation, including during ex vivo lung perfusion. Another potential use is detection of, targeted delivery to, and removal of injured or diseased tissue from various organs (e.g., gut polyps, lung fibrotic foci, etc.). The present invention may also be used for characterization of the mechanical properties of other organs and tissues, including gut, skin, vasculature, liver, etc. for research, diagnostic, prognostic, and therapeutic purposes. Mechanical evaluation of stem cell-tissue and cell-cell binding interactions is also enabled. A not-necessarily-final example of use is diagnosis and treatment of atherosclerosis. The device of the present invention can be used to measure the artery stiffness for patients prone to atherosclerosis and to remove the built-up fat, cholesterol, or calcium. Veterinary applications are also possible. Note however that such therapeutic or diagnostic methods do not fall within the scope of the claimed invention.

A method in accordance with the present invention can involve stiffness measurement of a tissue of interest that entails providing a probe having a compression head; locating the probe such that the compression head is proximate the tissue of interest; applying a pressure to the compression head; detecting a response at the tissue of interest in response to the pressure applied via the applying step; and calculating one or more physical properties of the tissue of interest based on the response. The method can be performed on the tissue of interest in in-vivo conditions, in which case the probe is inserted into a patient, or in ex-vivo conditions. For in-vivo applications, the tissue of interest can be imaged using an imaging element, which can be, for example, an optical fiber probe or a miniaturized camera. Additionally, ablation of damaged or otherwise problematic tissue can be performed with a laser localized on the probe. Furthermore, therapeutic compounds and/or fluorescent molecules can be delivered simultaneously to the tissue of interest. Note however that such therapeutic methods do not fall within the scope of the claimed invention.

In one example, the probe is introduced via a syringe needle proximate the tissue of interest. The probe can also be a balloon probe capable of being inflated to monitor its pressure and volume at the tissue of interest.

The method can also entail regulation of the pressure applied to the compression head (e.g., via a controller). In another example, the calculation step involves determining tissue stiffness. **In** another example, the tissue of interest can be a tumor, whose boundaries can be determined in real-time (e.g., via computer vision).

**In** another example, contact electrodes are placed proximate the tissue of interest and electrical resistance of the tissue of interest in response to the pressure is measured. **In** yet another example, the pressure is applied as suction force, and elongation length of the tissue of interest in response to the suction force is measured. **In** a still further example, the pressure is applied as compressive force, and tissue deformation length of the tissue of interest in response to the compressive force is measured. Both tissues or synthetic biomaterials can be evaluated using such methods.

**In** another example of the present invention, a device for evaluating stiffness of materials can be provided. The device can include a compression head; an imaging element coupled to the compression head; a motorized steering means adapted to move the imaging element and the compression head; a pressure network (e.g., a pressure line) adapted to apply positive or negative pressure to the compression head; and a controller adapted to regulate and control the pressure network.

In one example, the pressure line and imaging element are integrated with the motorized steering means as part of a steerable compartment of the device. The imaging element can be an optical fiber probe or a miniaturized camera. In one example, the controller is adapted to regulate pressure applied to the compression head, analyze collected tissue deformation data and calculate tissue stiffness. The inventive device also includes ablation means including a laser. In another example, the device also includes a delivery means for delivering therapeutic compounds to a tissue of interest. The delivery means can be further adapted to deliver fluorescent molecules. In one example, the device has a diameter less than 5 mm and a length of at least one meter.

In a further example, the inventive device has a balloon probe, adapted to be introduced via a syringe needle, wherein the balloon probe can be expanded to monitor pressure and volume at a tissue of interest. In certain applications, the inventive device can be adapted for use as an intraoperative tool to determine tumor boundaries in real-time. For instance, the controller can utilize computer vision to analyze the tissue of interest. For instance, the controller can be adapted to determine elongation length of the tissue of interest, the tissue deformation length of the tissue of interest, or the electrical resistance of the tissue of interest.

In additional examples, the compression head is a dome-shaped tip. The compression head can further include contact electrodes and a force sensor that monitor the compression force applied to the tissue of interest.

It is an object of the present invention to provide a minimally invasive probe capable of rapid and accurate quantification of tissue stiffness.

A second object of the present invention is to provide a probe that contains a motorized steerable compartment for minimally invasive insertion into the body.

It is another object of the present invention to provide a probe that is capable of applying a pressure network capable of providing negative or positive pressure to the tissue of interest.

It is yet another object of the present invention to provide an optical fiber probe that utilizes a miniaturized camera for guiding the navigation of the device and monitoring tissue deformation.

It is a further object of the present invention to provide a device that incorporates a computer-based controller that regulates the pressure, analyzes collected tissue deformation data, and calculates tissue stiffness.

It is an additional object of the present invention to provide a device that integrates the pressure line, imaging probes, and camera into the steerable compartment of the device.

It is yet another object of the present invention to provide a probe that enables stiffness measurements of internal tissues via an inflatable balloon needle.

It is another object of the present invention to provide a probe capable of (i) introducing fluorescent molecules to a target region for enhanced imaging and/or (ii) locally delivering therapeutics to the tissue of interest.

It is a not-necessarily final object of the present invention to provide a probe that enables the removal of tissue by laser ablation or biopsy.

### BRIEF DESCRIPTION OF THE DRAWINGS

For a more complete understanding of the present invention, reference is made to the following detailed description of various examples considered in conjunction with the accompanying drawings, in which:
FIG. 1 is a schematic of apparatus to measure local tissue stiffness;
FIG. 2A is a schematic illustration of insertion of the apparatus of FIG. 1 into a thoracic cavity;
FIG. 2B is a schematic illustration of video-based acquisition of elongation length data from the apparatus of FIG. 2A;
FIG. 2C is a table showing equations for calculating elastic modulus;
FIG. 3A is a schematic illustration of insertion of the apparatus of FIG. 1 into a respiratory tract;
FIG. 3B is a schematic illustration of video-based acquisition of elongation length data from the apparatus of FIG. 3A;
FIG. 4. is a schematic illustration showing measurement of tissue stiffness using a needle integrated with an expandable balloon;
FIG. 5A is a schematic illustration showing localized removal of tissue via biochemical treatment;
FIG. 5B is a schematic illustration showing localized removal of tissue via laser treatment;
FIGS. 6A-D involve (i) an overview of vacuum-based measurement of elastic moduli of soft biomaterials, showing a hydrogel (FIG. 6A), (ii) lung tissue deformed via vacuum pressure (FIG. 6B), (iii) a custom-built system to measure the stiffness of soft biomaterials (FIG. 6C), and (iv) a schematic of the measurement system of FIG. 6C (FIG. 6D);
FIG. 7A is a series of images showing a hydrogel undergoing stiffness measurement;
FIG. 7B is a merged image created from the images of FIG. 7A;
FIG. 8A is a series of graphs showing applied negative pressure and measured elongation length for various hydrogels undergoing cyclic vacuum-loading;
FIG. 8B is a series of graphs showing measured elongation length for various hydrogels undergoing the cyclic vacuum-loading process of FIG. 8A;
FIG. 8C is a series of graphs showing elastic moduli for the various hydrogels based on the measurements of FIGS. 8A and 8B;
FIG. 9A is a micrograph showing aerated gelatin;
FIG. 9B is a series of images showing the gelatin of FIG. 9A deformed under vacuum pressure;
FIG. 9C is a graph showing deformation of the aerated gelatin of FIG. 9A and a non-aerated control gelatin sample;
FIG. 9D is a graph showing elastic moduli for the control sample and aerated gelatin of FIG. 9C;
FIG. 10A is a photograph (i) and a schematic (ii) of a setup for measuring the stiffness of an ex vivo rat lung;
FIG. 10B is a series of images showing deformation under vacuum pressure of tissue from the lung of FIG. 10A;
FIG. 10C is a graph showing maximum elongation length of the lung tissue of FIG. 10B;
FIG. 10D is a graph showing elastic modulus of the lung tissue of FIG. 10B for various pressure values;
FIG. 10E is a force diagram showing tension (T) generated across the pleural surface of the lung tissue of FIGS. 10A and 10B due to increased P_{Alv} that leads to increased P_{V} required to stretch the lung tissue;
FIG. 11A is a schematic of lung injury induced by intratracheal instillation of trypsin with ICG fluorescent dye;
FIG. 11B is an image showing a photograph (i) and a NIR image (ii) of explanted rat lungs;
FIG. 11C is a graph showing pressure-volume curves of the rat lungs of FIG. 11B before and after injury that were obtained by measuring intra-alveolar pressure P_{Alv} and volume V_{L}; of air inspired or expired through the trachea of the lung using a small animal ventilator;
FIG. 11D is a series of H&E images of alveoli of the lungs of FIG. 11B, showing acute injured (i) and control (i.e., healthy) rat lungs (ii);
FIG. 11E is a graph illustrating maximum elongation length of the lungs of FIG. 11B;
FIG. 11F is a graph showing elastic moduli of the lungs of FIG. 11B under different P_{Alv} for both injured and control lungs;
FIG. 12A is photograph showing a custom-built imaging system for use with the optical imaging probe of the present invention;
FIG. 12B shows a front view imaging probe (12B(i)) and bright-field imaging (12B(ii)) and fluorescent imaging (12B(iii)) achieved using the probe of FIG. 12B(i);
FIG. 12C shows a side view imaging probe (12C(i)) along with fluorescent images obtained using the probe following injection of fluorescently labeled 10-um microparticles (12C(ii)) and fluorescently labeled mesenchymal stem cells (red) into the rat trachea (12C(iii));
FIG. 13A shows a photograph and a schematic diagram illustrating insertion of the front view imaging probe of FIG. 12B into a rat lung;
FIG. 13B is a pair of bright-field images obtained from the rat lung shown FIG. 13A;
FIG. 13C is a pair of fluorescent images obtained from the rat lung of FIG. 13A;
FIG. 14A is a 3D drawing of a motorized steerable catheter device (i), the steerable distal end (ii) of the device, and a schematic showing deflection of the distal end of the device achieved via servo motors and pulling wires integrated into the system (iii);
FIG. 14B is a photograph of a prototype of the motorized steerable catheter device of FIG. 14A;
FIG. 15 is a series of photographs illustrating vision-assisted tracking of a target using the prototype of FIG. 14B; and
FIGS. 16A-C involve a series of images showing insertion of the prototype device (see FIG. 14B) into the respiratory tract of explanted pig lung, including photographic images showing the process of device insertion into a plastic port connected to the trachea of the pig lung (FIG. 16A), a photograph of the prototype device placed into the pig lung airways (FIG. 16B), and a visualization of the airway interior using the prototype device inserted into the lung (FIG. 16C);
FIG. 17 is a series of diagrams illustrating the correlation between probe diameter and tissue elongation depth for a 0.58 mm probe (17A) and a 1.5 mm probe (17B);
FIG. 18 is a graph illustrating the effects of vacuum pressure on tissue elongation length;
FIG. 19 is a graph illustrating effects of rate change (S_{P}) of magnitude of the negative pressure (|P_{V}|) on tissue elongation length (L_{E});
FIG. 20 is a graph illustrating the correlation between vacuum pressure (|P_{V}|) and tissue elongation length (L_{E});
FIG. 21 is a force diagram showing tension force (*T*) within the tissue network and alveolar surface tension force (*F*ST) at the air-liquid interface where increasing *P*_{Alv} results in elevated *T* and *F*_{ST} leading to greater *P*_{V} needed to stretch the lung parenchymal tissue;
FIG. 22 is a series of photographs illustrating lung pleura integrity for healthy (22A) and trypsinized (22B) rat lungs;
FIG. 23 is a schematic representation of a compression-based tissue palpation device and its operation procedure (23A) and a photograph (23B) showing the distal end of a prototype of a compression-based stiffness measurement device;
FIG. 24 is a photograph of a steerable compression-based tissue stiffness measurement device;
FIG. 25A is a schematic of the electrical circuit used to measure the force in the form of voltage (*V*);
FIG. 25B is a series of Calibration curves that correlate the measured voltage (*V*) and force (*F*) using different resistance values (*R*);
FIG. 26 is an experimental setup showing a test of the compression-based tissue stiffness measurement device for measuring the stiffness of isolated porcine lung;
FIG. 27A is a schematic and graphs showing measurement of voltage and electrical current via a force sensor and contact electrodes, respectively, to determine the tissue stiffness; and
FIG. 27B is a table illustrating calculation of stiffness measurement results of porcine lung tested using a compression-based device via measured electrical signals.

### DETAILED DESCRIPTION

The following disclosure is presented to provide an illustration of the general principles of the present invention and is not meant to limit, in any way, the inventive concepts contained herein. The scope of the invention is defined by the appended claims. Moreover, the particular features described in this section can be used in combination with the other described features in each of the multitude of possible permutations and combinations contained herein.

All terms defined herein should be afforded their broadest possible interpretation, including any implied meanings as dictated by a reading of the specification as well as any words that a person having skill in the art and/or a dictionary, treatise, or similar authority would assign thereto.

Further, it should be noted that, as recited herein, the singular forms "a", "an", "the", and "one" include the plural referents unless otherwise stated. Additionally, the terms "comprises" and "comprising" when used herein specify that certain features are present in that example, however, this phrase should not be interpreted to preclude the presence or addition of additional steps, operations, features, components, and/or groups thereof.

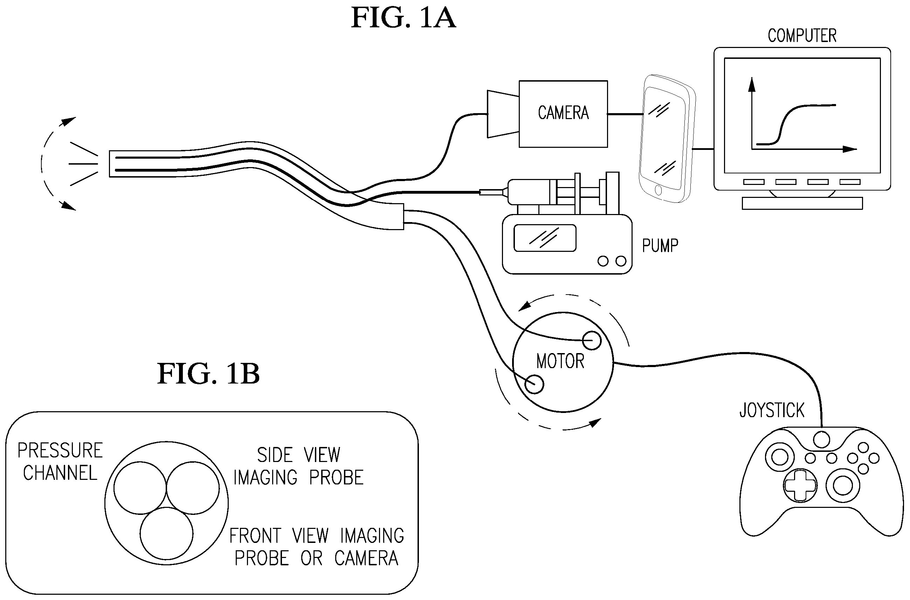

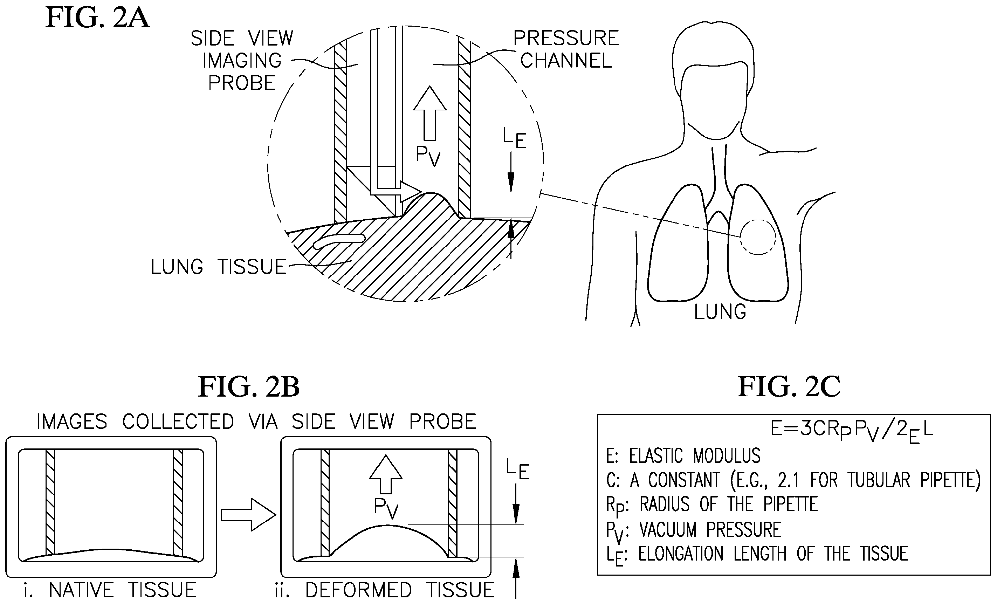

All examples and conditional language recited herein are intended for pedagogical purposes to aid the reader in understanding the principles of the invention and the concepts contributed by the inventor to furthering the art, and are to be construed as being without limitation to such specifically recited examples and conditions. The present invention relates to a locally deployable device that is capable of non-destructive and rapid measurement of tissue and organ stiffness (see FIG. 1). In one example, it is a pressure-assisted steerable device that can measure stiffness (i.e., elasticity) of local tissue within the patient's body in a localized and minimally invasive manner. A pressure channel, imaging probe(s), and camera are integrated into a steerable compartment of the device. The device is comprised of: i) a motorized steerable compartment that can be inserted minimally invasively into a patient's body; ii) a pressure network that can apply negative or positive pressure to the tissue being evaluated; iii) optical fiber probe(s) and a miniaturized camera that can guide navigation of the device and monitor the tissue deformation; and iv) a computer-based controller that regulates the pressure, analyzes collected tissue deformation data, and calculates tissue stiffness. The pressure line, imaging probe(s), and camera are integrated into the steerable compartment of the device where the tissue stiffness measurement is achieved by contacting the tissue surface of interest with the device tip. Specifically, negative pressure (PV) created by a vacuum pump is applied directly to the tissue surface via the device tip while the elongation length of the tissue (LE) can be determined via the side view imaging probe (see FIGS. 2A-2B).

A device can be inserted the thoracic cavity via a small incision (diameter: < 1 cm) created in the chest, and the device tip is placed directly onto the lung pleura surface. Negative pressure (*P*V) is then applied via pressure channel while deformation of the lung tissue is continuously monitored via side view imaging probe. Elongation length (*L*E) of the deformed tissue is determined from the video acquired in real time. The elastic modulus (E) of the biomaterial, which is a quantity that measures the tissue stiffness, is then determined by the formula E = 3C*RP*PV/2πLE, where C is a constant specific to the geometry of the pipette used to apply vacuum pressure to the samples, RP is the radius of the pipette used for measurement, PV is the vacuum pressure, and LE is the elongation length of the samples. For a tubular pipette, a typical value for C is ~2.1 (see FIG. 2C).

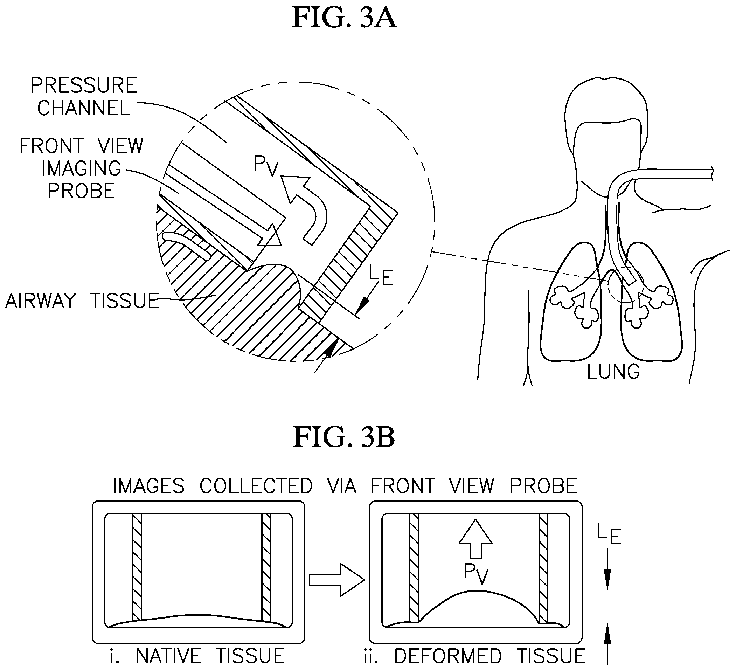

To measure luminal tissue, such as the tissues within the respiratory, gastrointestinal, or urinary tracts, the device is designed and created in a way that vacuum pressure is provided to the local tissue from the circumferential surface of the device tip (see FIG. 3A). Deformation and elongation of the tissue can be monitored via a front view imaging probe integrated into the device (see FIG. 3B). The device can be inserted into the respiratory tract via patient's mouth or nose and the device tip is positioned onto measurement site. Negative pressure is then applied while deformation of the tissue is continuously monitored via front view imaging probe. Elongation length (*L*E) of the deformed tissue is determined from the video acquired in real time. Elastic modulus, which is a quantity that measures the tissue stiffness is calculated using an equation shown in FIG. 2C.

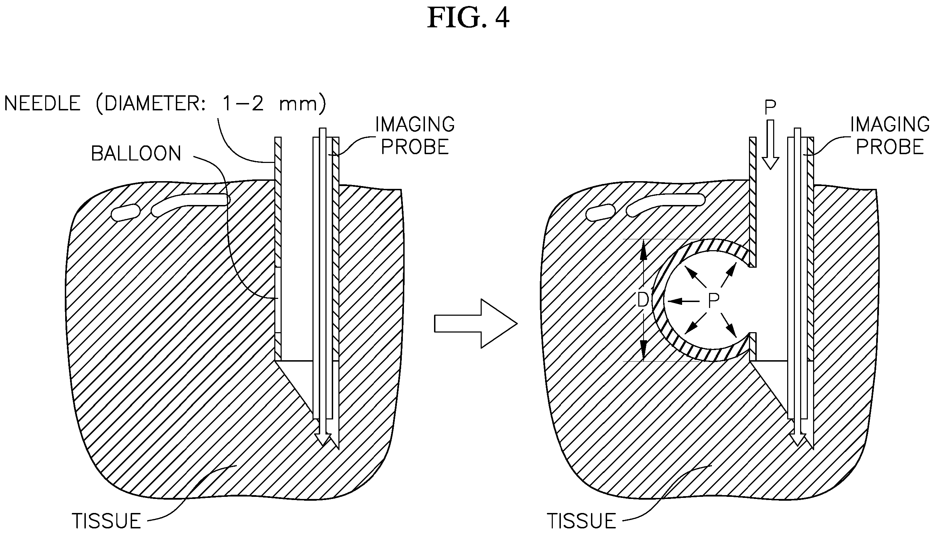

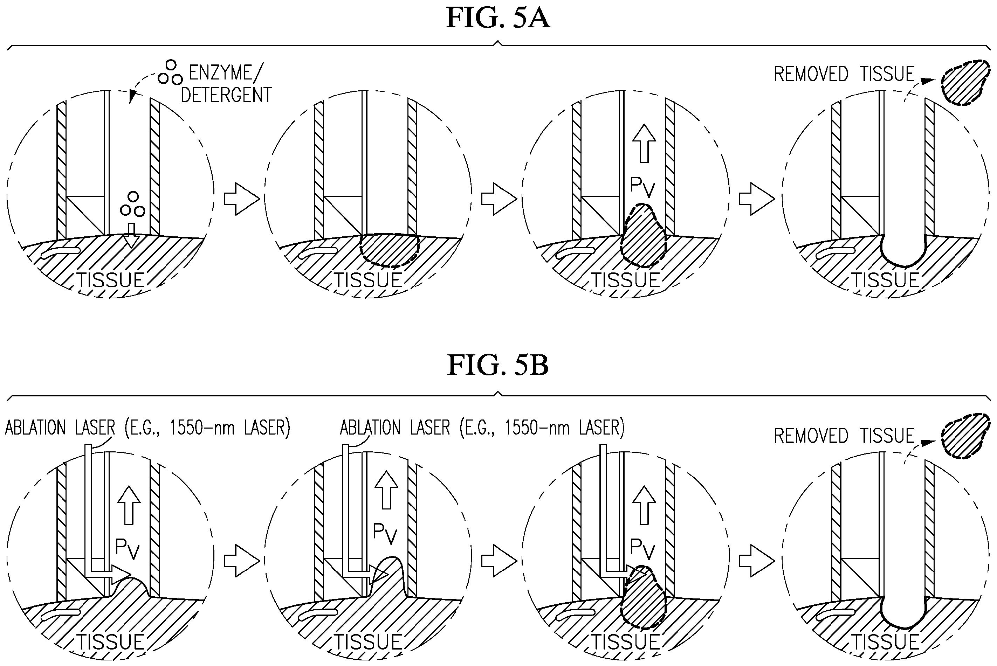

In addition, to measure stiffness of the internal tissue or biomaterial, a syringe needle (e.g., diameter: 1-2 mm) integrated with a balloon can be inserted locally (see FIG. 4). Specifically, in one example, a syringe needle integrated with a balloon can be expanded via pressurized air or fluid is inserted into tissue to measure internal tissue stiffness. In this approach, the tissue stiffness (*E*) is obtained by relating the pressure of air or fluid (*P*) and the diameter of the balloon (*D*)*. P* is measured using an air or liquid pressure sensor externally. *D* is estimated from the volume (*V*) of air or fluid introduced into the syringe where *V* = 1/6πD³. While the balloon-needle is positioned internally, air or fluid (e.g., water) is provided to expand the balloon. Pressure (P) and volume (V) of the air or fluid are measured using sensors externally. Elastic modulus (E) of the tissue or biomaterial is calculated using P and V. Furthermore, the device can be used to locally deliver therapeutics to a pathologic region; take a tissue sample for in vitro analysis; and remove diseased or injured tissue. For example, fluorescent molecules (e.g., antibodies, small molecules, etc.) can be introduced via the pressure channel in order to visualize particular tissue features, such as tumors. Local tissue can also be disrupted by introducing biochemical agents (e.g., proteolytic enzymes or detergent molecules), whereby the disrupted tissue can be removed through application of vacuum pressure to the pressure channel (see FIG. 5A). In some examples, proteolytic enzymes or detergent (e.g., SDS, CHAPS) is locally introduced to disrupt tissue where the disrupted tissue is removed by applying negative pressure (PV). In other examples, local tissue can be removed by an illuminating laser (e.g., a 1550-nm laser) that can thermally ablate the tissue while the tissue is being stretched via the applied vacuum pressure (FIG. 5B). Specifically, in the context of the claimed invention, laser-based ablation means are included in the stiffness evaluating device.

### Example 1: Vacuum-enabled stiffness measurements of rat lung tissue and soft hydrogels.

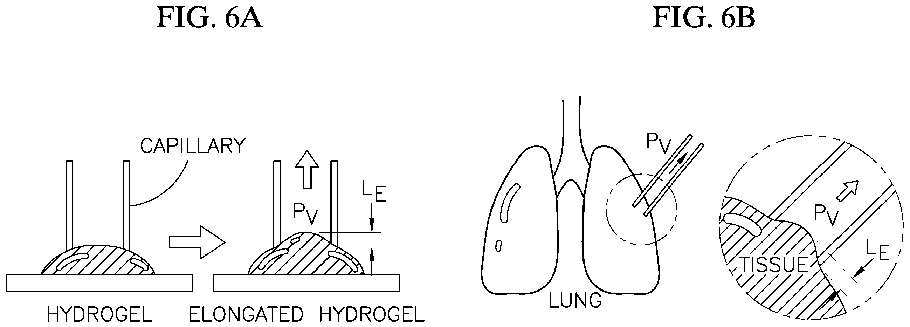

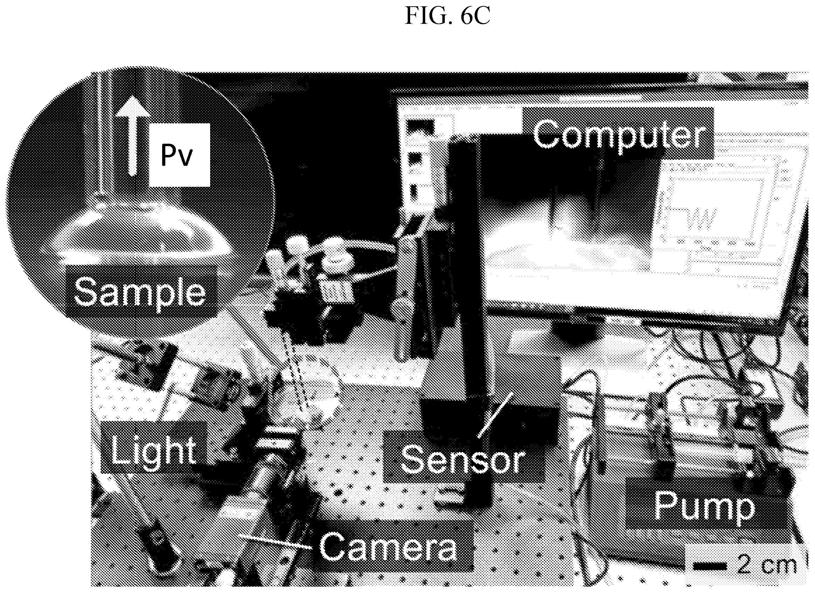

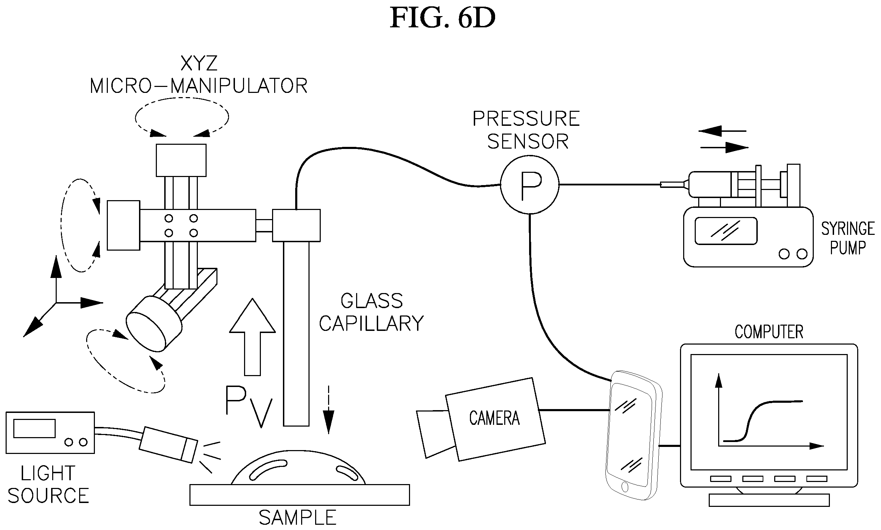

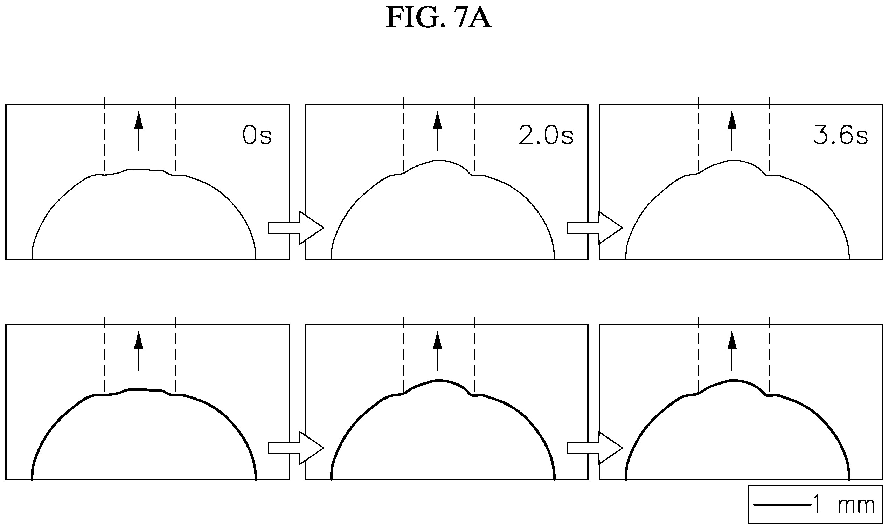

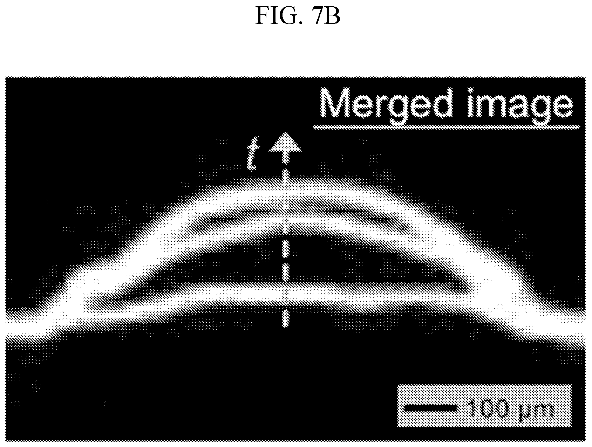

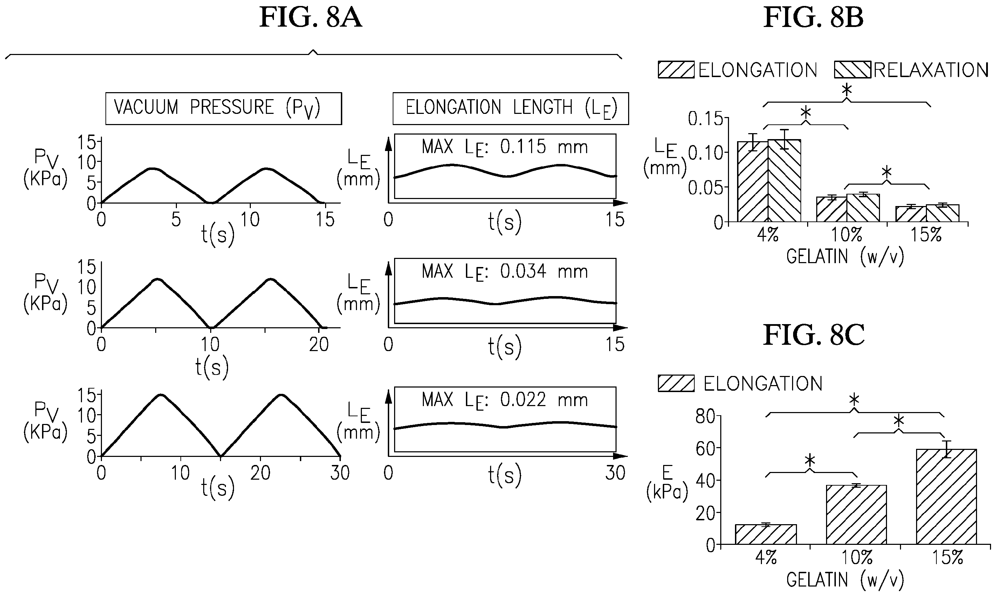

A vacuum-based platform for measurements of elastic moduli of lung tissues and soft biomaterials, such as gelatin hydrogels (FIGS. 6A-D) was developed. Functionality of this platform was tested by using a dome-shaped 4% gelatin hydrogel formed on a substrate (FIG. 7). Stiffness measurement was demonstrated using a dome-shaped 4% w/v gelatin hydrogel formed on a PDMS substrate. To enhance visibility of gel deformation, fluorescein dye was added to the gelatin. Via edge detection, the deformed shape of the gelatin was determined. Negative pressure was applied to the hydrogel via a glass capillary (inner diameter: 0.58 mm) that was gently placed on top of the gel. When the gel was exposed to -8.2 kPa, it elongated 0.115 mm. To accurately determine the deformed shape of the hydrogel, the boundary of the gel was determined via image processing, and in particular, a Canny Edge detector (FIGS. 7A-B). FIG. 7B shows a merged image of the deformed region of the gelatin showing elongation of the gel due to vacuum pressure over time. Also, gelatin hydrogels with different concentrations (i.e., 4%, 10%, and 15%) were prepared to investigate whether the platform can determine the stiffness of the hydrogels with different mechanical properties (FIGS. 8A-C). When cyclic negative pressure was applied to the gels, different elongation lengths were observed in the gels (FIG. 8A). For all samples, the rate of negative pressure increased or decreased (i.e., slope of aspiration curve) was approximately 2.25 kPa/s.

Based on the experimental parameters, such as negative pressure and tube diameter, the elongation lengths measured from the hydrogels allowed for the determination of the elastic modulus of different gels, wherein the calculated values agreed well with the values reported in the literature. For 4%, 10%, and 15% gelatins, E values were 11.913 ± 1.008, 36.568 ± 1.297, and 59.108 ± 3.932 kPa, respectively (FIGS. 8B-C). Elongation length (*LE*) of the gelatin under *P*V of -5 kPa was determined during elongation and relaxation period of the gel, respectively. Elastic modulus (*E*) of each gelatin hydrogel was determined based on the measurement condition. All values represent mean ± standard deviation. **p* < 0.001

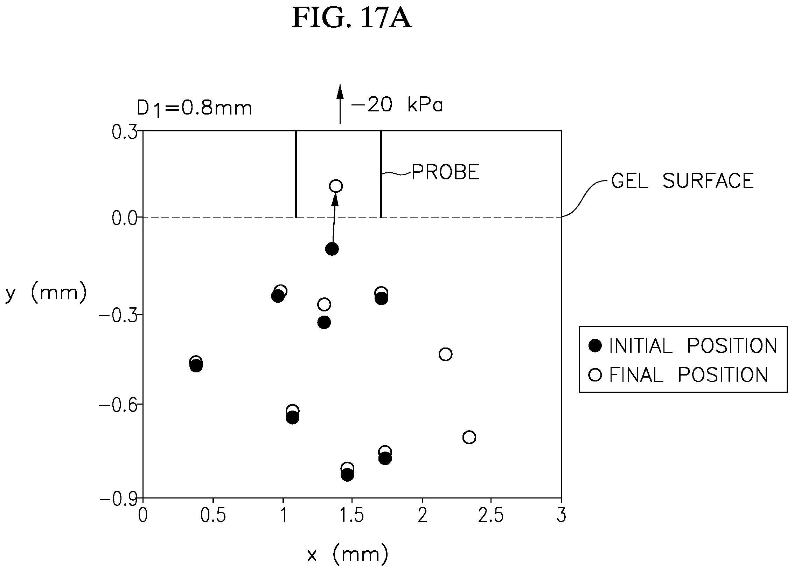

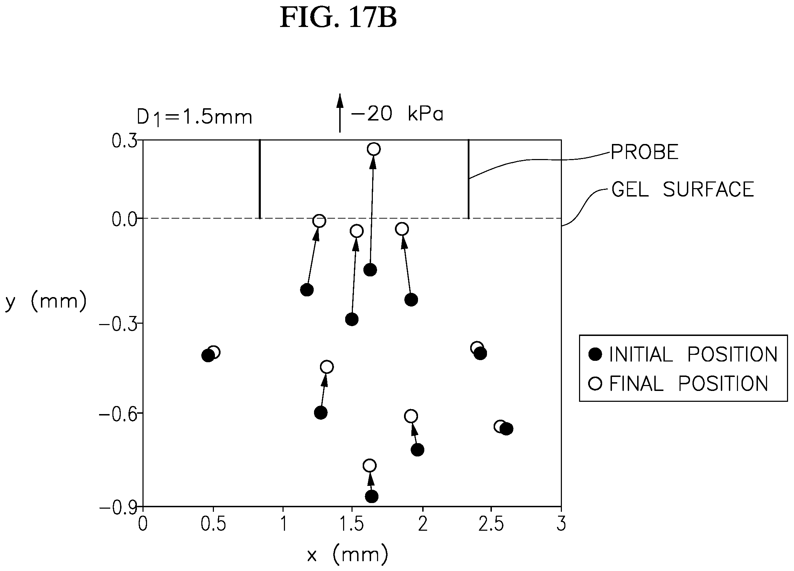

Comparisons of moduli between different gelatin groups (e.g., 4% vs 10%, 4% vs 10%, etc.) indicated significant correlation between *E* and gelatin concentration (*p <* 0.001). Notably, inner diameter of the capillary probe (*D*_{I}) was proportional to the depth of the negative pressure energy propagated within the gel (FIGS. 17A and 17B). In particular, a larger probe (*D*_{I} = 1.5 mm) can elongate and therefore, measure stiffness of deeper regions of hydrogel compared to a thinner probe (*D*_{I} = 0.58 mm) which provides more superficial measurements.

Specifically, the probes were used to investigate correlation between the tube diameter and the depth of propagation of the vacuum pressure energy within a gelatin hydrogel. To visualize elongation of the hydrogel, 10-µm fluorescent particles were mixed in a 4% gelatin hydrogel and their movements were monitored using a camera. under a constant vacuum pressure of -20 kPa, the larger probe (d1 =1.5mm) was able to cause movement of particles located deeper within the gel block, suggesting larger probe can allow measurement of more centrally located tissues.

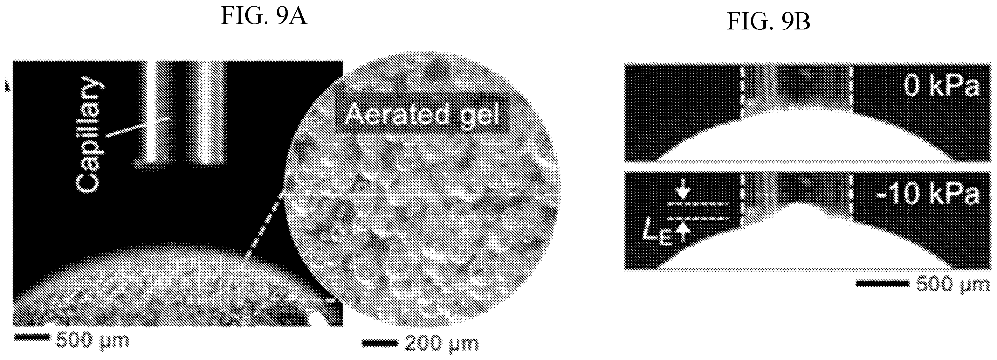

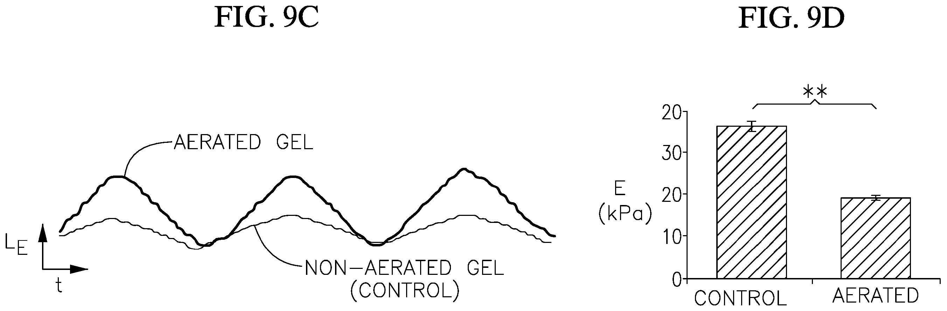

This platform was used to measure the stiffness of porous gelatin hydrogels that mimic aerated lung tissue (FIG. 9). It was then determined that the stiffness of porous 10% gelatin (E = 19.065 ± 1.298 kPa) was substantially lower than solid 10% gelatin (E = 36.568 ± 1.297 kPa) (FIGS. 9A-D). Aerated 10% w/v gelatin (FIG. 9A) was tested to investigate the correlation between gel porosity and stiffness. FIG. 9B shows images of the aerated gel showing deformed shape under vacuum pressure (*P*V) of -10 kPa. Deformation of both aerated and nonaerated (control) 10% w/v gelatin under cyclic vacuum pressure loading over time is shown in FIG. 9C. FIG. 9D shows elastic modulus E of the aerated and non-aerated 10% w/v gelatin. All values represent mean ± standard deviation. **p* < 0.001.

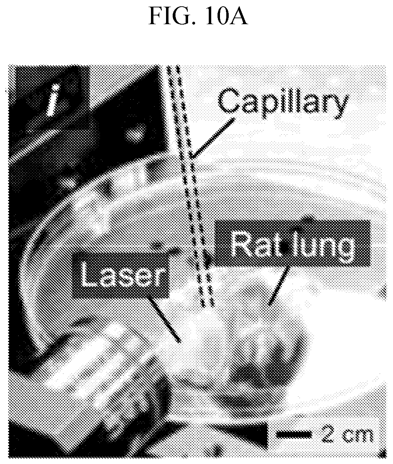

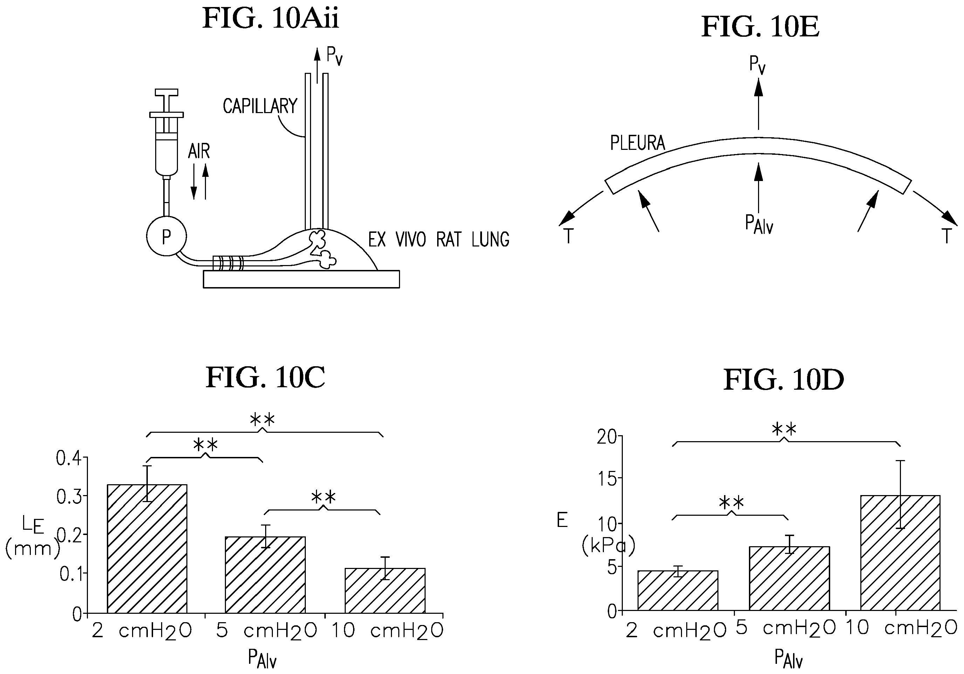

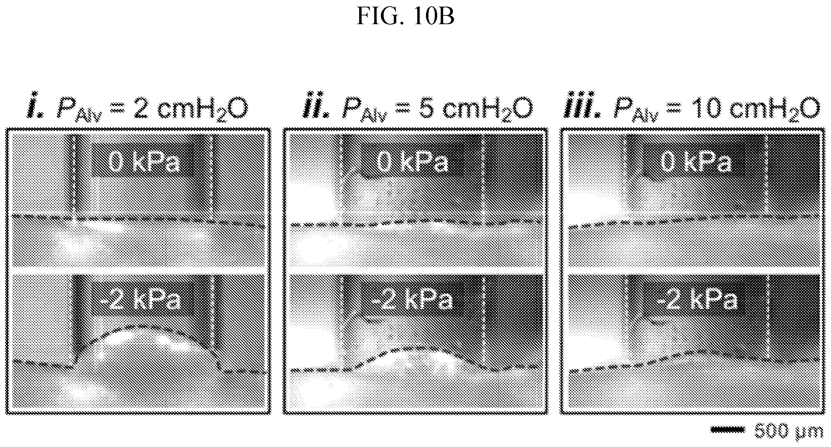

Elastic behaviors of rat lung tissues (FIGS. 10A-E) were then investigated. To improve the visibility of the tissue, the lung tissue was labeled with fluoresceine fluorophore prior to measurements (FIG. 10A; P: air pressure sensor). Notably, elongation length (LE) of the lung tissue was dependent on the pressure inside the lung (PALV). As a result, for PALV values of 2, 5, and 10 cmH2O, E values were determined to be 4.443 ± 0.613, 7.420 ± 1.056, and 13.174 ± 3.854 kPa, respectively (FIGS. 10B-E). In FIG. 10B, lung tissue was subjected to vacuum pressure of 2 kPa via a glass capillary tube (inner diameter = 1.5 mm) while the intra-alveolar pressure (P_{Alv}) was maintained at different pressure levels: (i) 2 cmH₂O (ii): 5 cmH₂O, (iii) 10 cmH₂O. FIGS. 10C and 10D illustrate, respectively, maximum elongation length (L_{E}) and elastic modulus E of lung tissue against different P_{Alv}. All values in FIG. 10 represent mean +/- standard deviation; **p < 0.05.

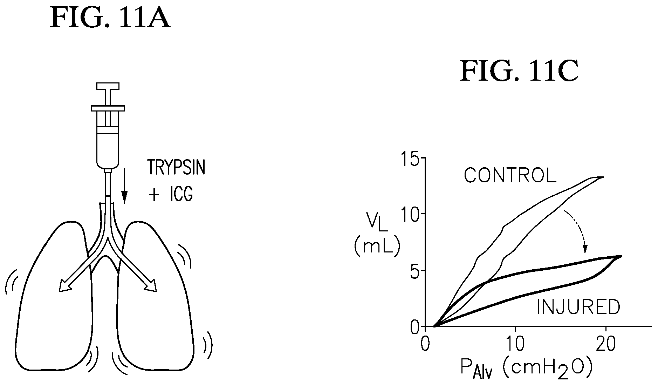

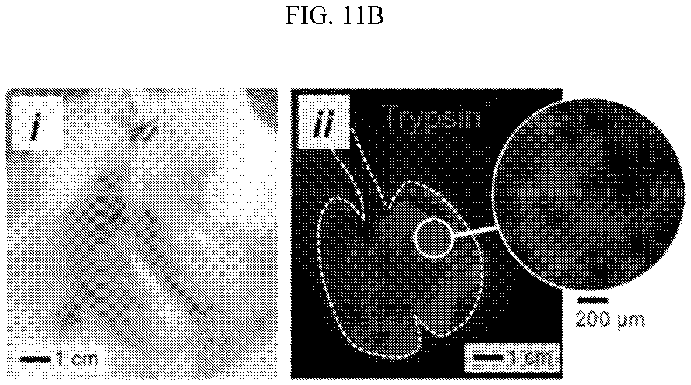

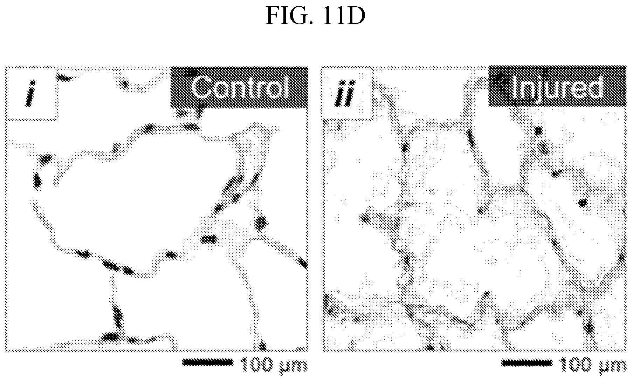

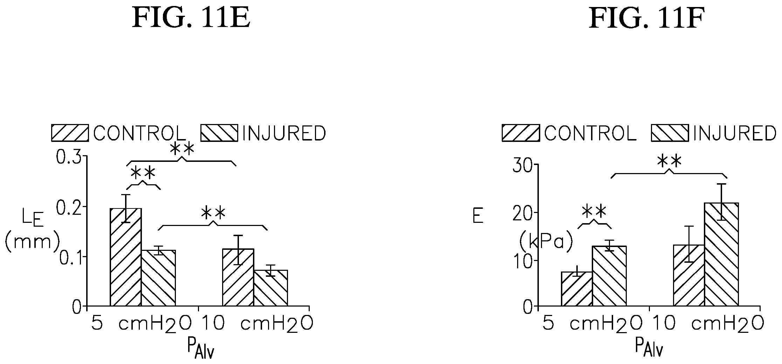

In addition, the measurement platform allowed for the quantification of the differences between the healthy and injured lung in their tissue stiffness (FIG. 11). Acute lung injury was induced by instilling 0.25% trypsin solution through the trachea of the rat lung. To visually confirm delivery of the solution in the lung, indocyanine green (ICG) fluorescent dye molecules were added to the trypsin solution (FIGS. 11A-B). FIG. 11B shows (i) explanted rat lungs before injury and (ii) distribution of trypsin/ICG (red) visualized via NIR imaging. FIG. 11C shows pressure-volume (PV) curves of the lung before and after injury, which were obtained by measuring the intra-alveolar pressure (P_{Alv}) and volume (V_{L}) of air inspired or expired through the trachea of the lung using a small animal ventilator. The compliance of the injured lung substantially decreased (FIG. 11C), while histologic analysis of the injured tissue showed severe damage in the alveolar tissues (FIG. 11D). Notably, stiffness of the injured lung was greater than healthy lung (FIGS. 11E-F). The increase in stiffness of the injured lung is likely due to dysregulation of native pulmonary surfactant in the alveoli caused by displacement and dilution by the enzyme solution. All values in FIG. 11 represent mean +/- standard deviation; **p < 0.05.

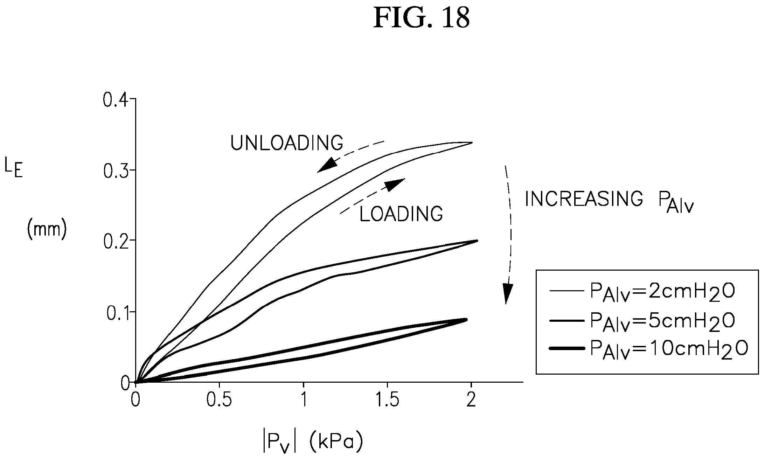

Intact rat lungs were also used to investigate whether the elastic modulus of lung tissue can be measured non-destructively using the vacuum-assisted method. During measurements, the intra-alveolar pressure of the lung (*P*_{Alv}) was maintained at a constant level (2, 5, 10 cmH₂O) without ventilating lungs to minimize motion-induced measurement error and prevent tissue damage that could be caused by uncontrolled contact of the capillary probe with the tissue surface. To improve visualization of tissue deformation, a 488-nm laser was used to directly illuminate the lung pleural tissue labeled with fluoresceine molecules prior to measurements. The pressure inside the lung (*P*_{Alv}) was controlled and monitored using a syringe connected to a pressure sensor. Photographs showed deformation of lung tissue under a negative pressure (*P*_{V}) of -2 kPa via a capillary tube (inner diameter: 1.5 mm) while different *P*_{Alv} (2, 5, 10 cm H₂O) were maintained within the lung. Further, the elongation length of the lung tissue (*L*_{E}) was measured, while *P*_{V} was increased and decreased to imitate the stress-strain (i.e., pressure-volume) measurements of lung (FIG. 18) measured *L*_{E} varied nonlinearly in response to *P*_{V} while the loading-unloading curve exhibited hysteresis which is a unique behavior of viscoelastic materials, including lung tissue.

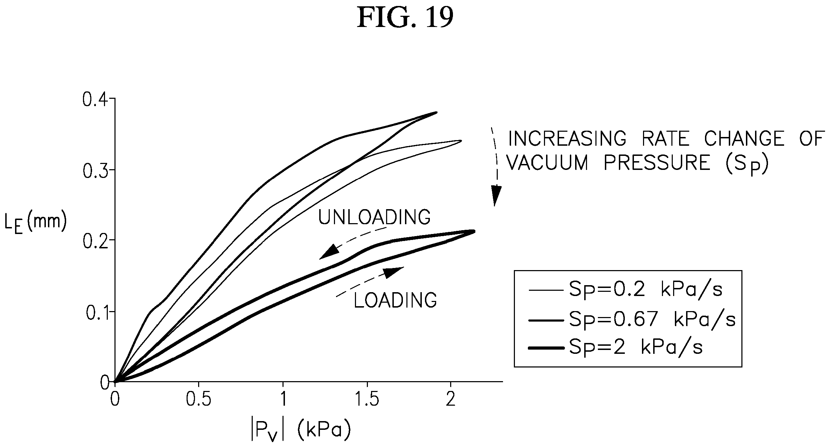

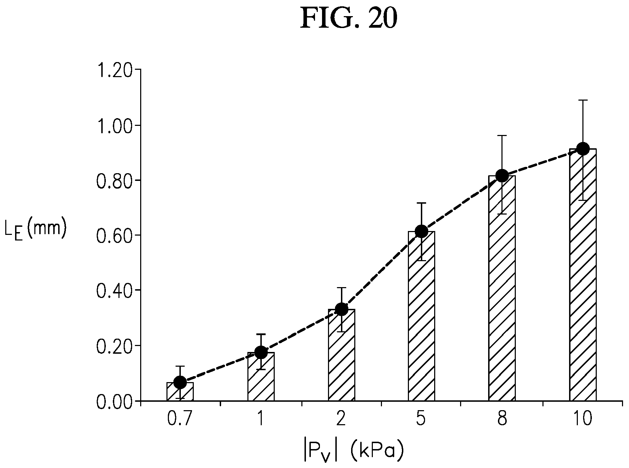

FIG. 18 also shows the effects of vacuum pressure (P_{Alv}) on tissue elongation length (L_{E}): for rat lung maintained at different P_{Alv}, L_{E} were measured continuously while P were varied between 0 and 2 kPa. L_{E} and P_{V} were nonlinearly related and the curves exhibited hysteresis. Increasing P_{Alv} resulted in reduced L_{E} due to increased forces within the alveolar network and pleural layer. In addition, *L*_{E} was measured while varying rate of change of vacuum pressure magnitude (|*P*_{V}|) (i.e., 2 kPa/s, 0.66 kPa/s, and 0.2 kPa/s) (FIG. 19). Notably, increased rate change of |*P*V| resulted in reduced *L*_{E} indicating viscoelastic deformation pattern of the lung. It was further confirmed that the non-linear deformation of lung tissue was present by measuring *L*_{E} while increasing |*P*_{V}| from 0.7 to 10 kPa in a step-wise manner (FIG. 20). At lower pressure (|*P*_{V}| < 2 kPa), *L*_{E} increased rapidly with |*P*_{V}|, while further increasing magnitude of the negative pressure (e.g., |*P*V| > 5 kPa) resulted in decreased rate of tissue elongation, highlighting non-linear correlation between *L*_{E} and |*P*_{V}|.

With reference to FIG. 19, effects of the rate change (S_{P}) of magnitude of the negative pressure (|P_{V}|) on tissue length (L_{E}) is shown. L_{E} were continuously recorded while varying (|P_{V}|) between 0 and 2 kPa. The obtained curves showed a nonlinear relationship between L_{E} and (|P_{V}|) and hysteresis was observed as L_{E} were generally greater during unloading than loading. Furthermore, changing the pressure values rapidly (i.e., increasing S_{P}) resulted in reduced overall L_{E}. Together, the results confirmed viscoelastic behavior of the lung parenchymal tissue.

With reference to FIG. 20, the correlation between vacuum pressure |P_{V}| and tissue elongation length (L_{E}) is illustrated. L_{E} were measured against different |P_{V}| between 0.7 and 10 kPa. While L_{E} and |P_{V}| were approximately linearly related below 2 kPa, L_{E} increased slowly at higher |P_{V}| (e.g., above 5 kPa) suggesting that greater negative pressure is needed to deform load-bearing lung tissue. Because of this nonlinear relationship between L_{E} and |P_{V}||P_{V}| =2 kPa was used throughout the tests to compare stiffness of different lungs.

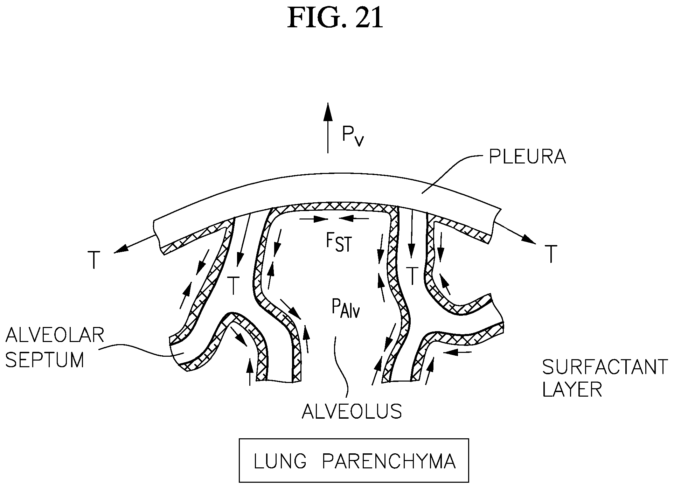

Notably, as pressure inside the lung increased, *L*_{E} decreased. For *P*_{Alv} of 2, 5, and 10 cmH₂O, *L*_{E} were 0.33 ± 0.05, 0.20 ± 0.03, and 0.12 ± 0.03 mm and *E* were 4.4 ± 0.6, 7.4 ± 1.1, and 13.2 ± 3.9 kPa, respectively. The results indicate that there was an interaction between *P*_{Alv} and *E* (0.2 vs 0.5 kPa: *p =* 0.005; 0.2 vs 1.0 kPa: *p* = 0.0058; 0.5 vs 1.0 kPa: *p =* 0.067). Such alveolar pressure dependence is due to the changes in tension (*T*) within the pleural layer and alveolar septal network with respect to the air pressure inside. As the lung volume increases, the collagen fibrils in the tissue become stress-bearing while their waviness is lost, leading to increased tissue elastic modulus. Therefore, as the internal pressure of the lung elevates by increasing the volume of air in the lung, tension in the lung tissue also increases, requiring a greater vacuum pressure to stretch the lung tissue against the resisting tensile force (FIG. 21) Lung stiffness measured using the vacuum-based method were within ranges reported in the literature, obtained using a broad range of mechanical testing methods, including indentation, atomic force microscopy, and tensile testing.

### Example 2: Optical Fiber Imaging Probe for Monitoring Tissues

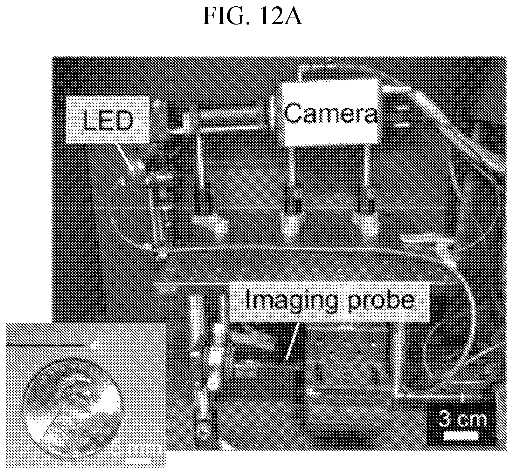

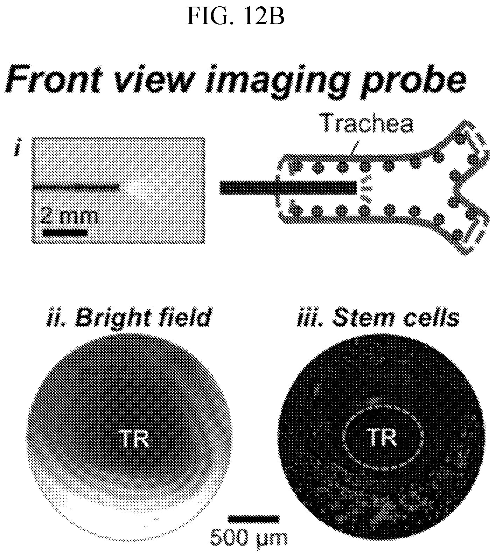

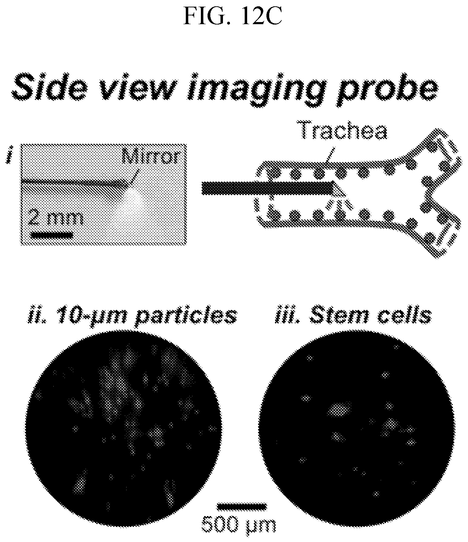

A custom-built imaging platform was created utilizing optical imaging probes (both front view and side view probes) that were capable of imaging local tissues in bright-field and fluorescence (FIG. 12). The imaging system is comprised of a scientific camera, an LED or laser light source, optical filters, and an optical imaging probe (FIG. 12A). Using this imaging system, visualization of the interior of the rat lung was demonstrated. In particular, both bright-field and fluorescent imaging of the inside of the rat trachea were achieved using a front view imaging probe (FIG. 12B). Using a side view imaging probe, the luminal surface of the rat trachea was visualized with reduced optical distortion (FIG. 12C).

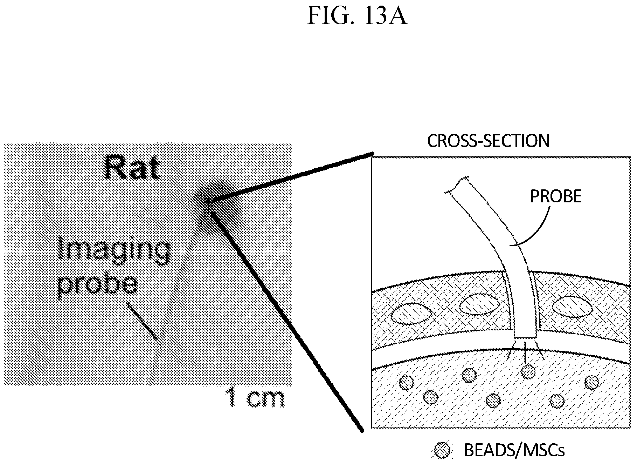

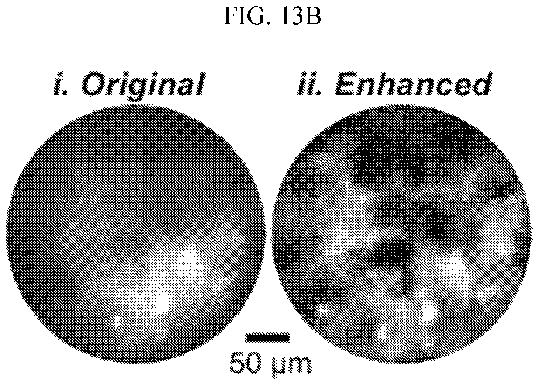

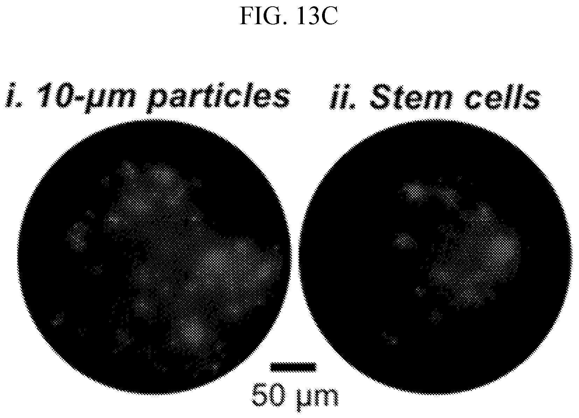

For fluorescent imaging using both imaging probes, red 10-µm microparticles or mesenchymal stem cells were implanted. In addition, this imaging system was used to visualize the rat lung in situ (FIG. 13). Imaging of the rat lung was achieved by inserting the probe into the thoracic cavity through a small incision created in the chest of the animal (FIG. 13A). Bright-field images of the rat lung were obtained through this imaging approach, wherein a front view imaging probe was placed near the lung pleura. By enhancing the contrast of the image, individual alveoli could be visualized clearly (FIG. 13B). Furthermore, this imaging system allowed fluorescent imaging of the rat lung in situ wherein the 10-µm particles and mesenchymal stem cells (red) introduced into the alveolar space of the rat lung were clearly visualized (FIG. 13C) through the thin pleural layer. The results suggest that this imaging system can be used to locally inspect tissue deformation and measure elongation of the deformed tissue via vacuum pressure.

### Example 3: Construction of a Steerable Catheter for Integration of Vacuum Channel and Imaging Probe

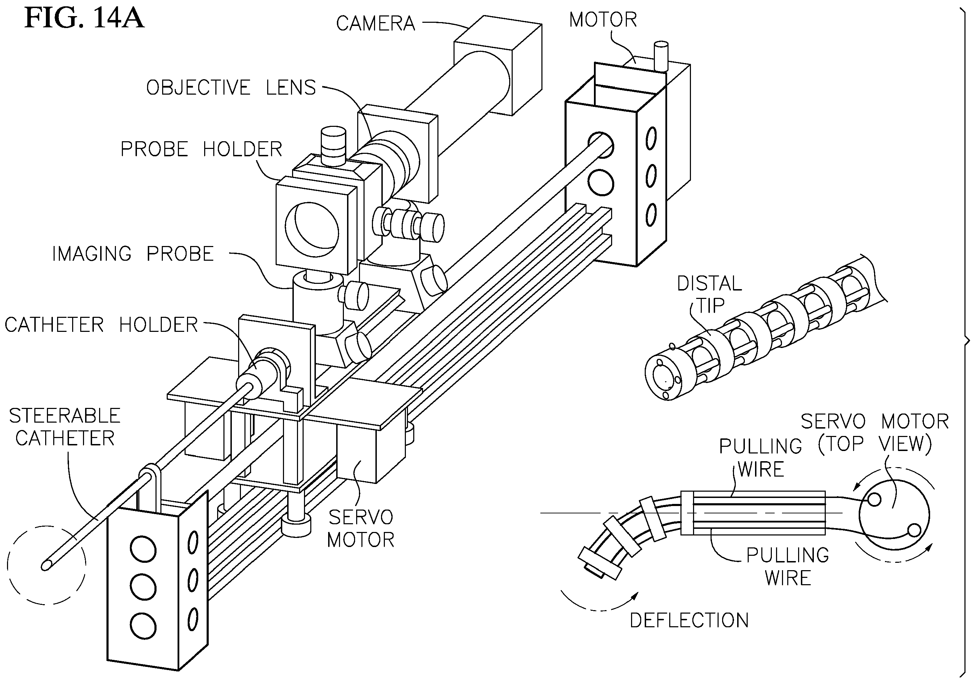

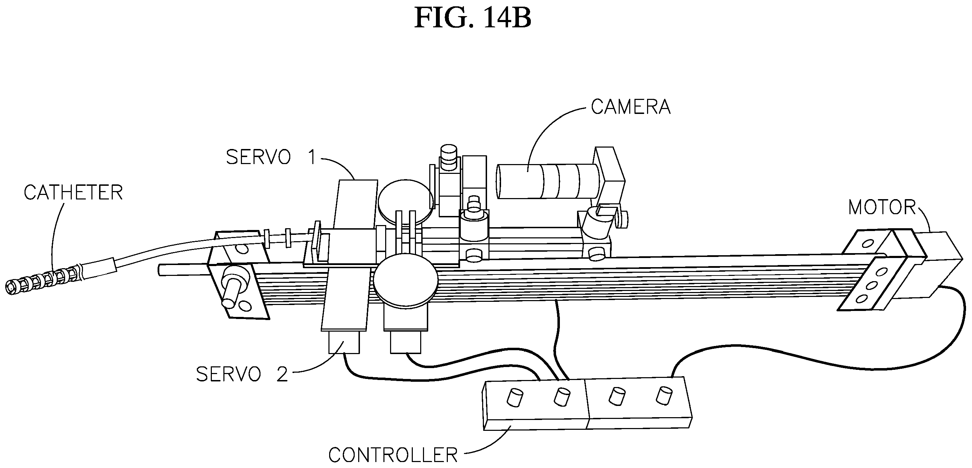

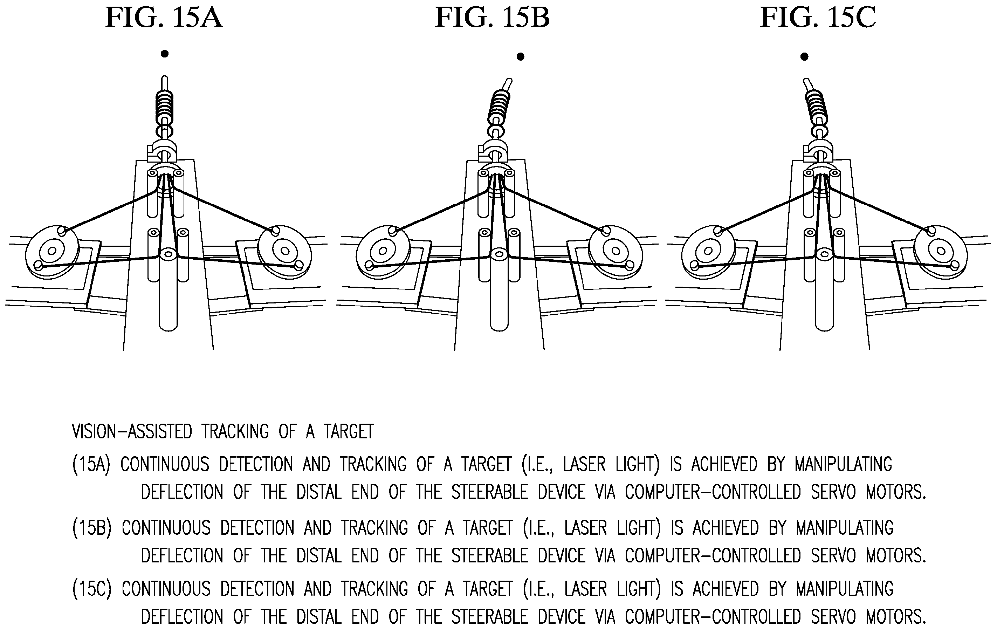

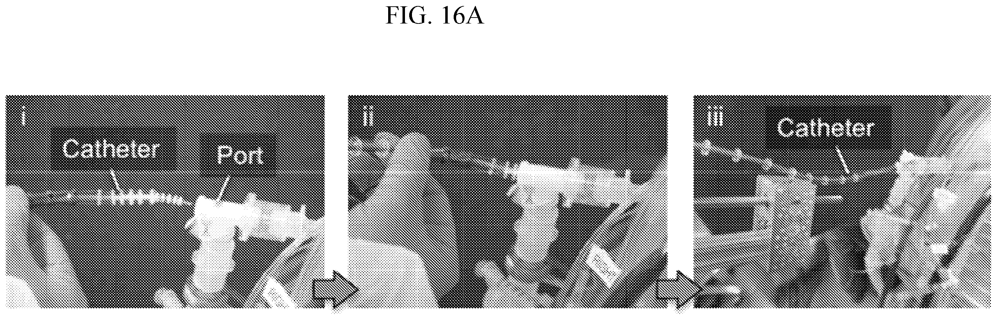

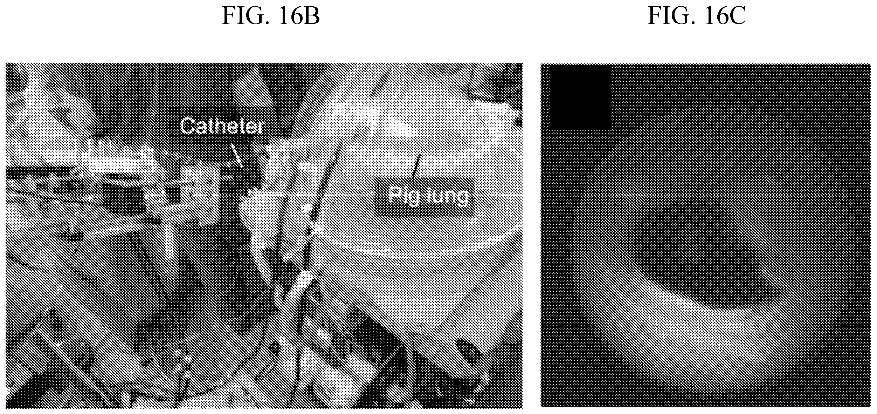

A motorized steerable catheter device was created, into which the vacuum channel and imaging probe can be integrated for localized tissue stiffness measurements (FIG. 14). As shown in the 3D rendering image of the device, servo motors, pulling wires, and a motor controller integrated together collectively control deflection and translational movement of the device tip (FIG. 14A). A protype of the device was constructed, which comprises three servo motors, a motor controller, and an optical fiber imaging probe that is capable of vision-assisted three-dimensional navigation in space (FIG. 14B) In the prototype, the steerable catheter is integrated with servo motors and a motor controller (i.e., joystick) that can control movements of the device. Using this device, vision-assisted continuous tracking of an object was demonstrated by manipulating deflection movements of the device using a motor controller via computer-controlled servo motors (FIGS. 15A-C). Furthermore, the feasibility of the device was demonstrated in visualization of the airway lumen using explanted swine lungs (FIGS. 16A-C). Via an access port, the device was inserted into the lung through the trachea (FIGS. 16A-B). The interior of the airway of the lung was visualized using the device inserted (FIG. 16C).

The invention described herein facilitates simultaneous tissue evaluation and removal. While similar technologies/devices are limited to assessment of tissue stiffness, the present invention can allow not only localized tissue evaluation, but also tissue biopsy or ablation (FIG. 5). The multi-functional approach of the present invention can reduce the number of procedures required for the patients by allowing simultaneous diagnosis and treatment during a single intervention.

Regarding computer vision-assisted accurate determination of tissue deformation, it is important to determine the exact deformed shape of the tissue caused by exposure to vacuum pressure. In the inventive device, the shape of the tissue before and after the vacuum-induced deformation can be accurately determined in real time via computer-vision enabled boundary detection (or edge detection) methods (FIGS. 7-8). The computer-assisted tissue boundary detection can allow accurate measurement of the tissue elongation from which stiffness of the tissue being evaluated can be calculated.

Regarding in situ fluorescence tissue visualization, to enhance the quality of images acquired during tissue deformation for accurate assessment of tissue deformation, the present invention can be integrated with in situ fluorescence imaging capability (FIGS. 6A-D), wherein *P*V: vacuum pressure. *L*E: elongation length, and P: Air pressure sensor. Due to autofluorescence, human tissues normally emit green lights when they are exposed to blue light. Accordingly, the local tissue being evaluated using the present device is illuminated with blue laser light (wavelength of approximately 488 nm), while green light generated by the tissue is collected via a camera connected to the device externally. The acquired images are processed via computer algorithms to determine the tissue boundary, elongation length, and tissue stiffness. In addition, fluorescent molecules (e.g., fluoresceine, rhodamine, tagged antibodies) that can label the tissue and/or specific tissue features fluorescently can be introduced through the device lumen to further improve the visibility of the tissue and identify particular tissue features and enhance the accuracy of tissue deformation and elongation.

### Example 4: Alterations in Stiffness Caused by Alveolar Disruption

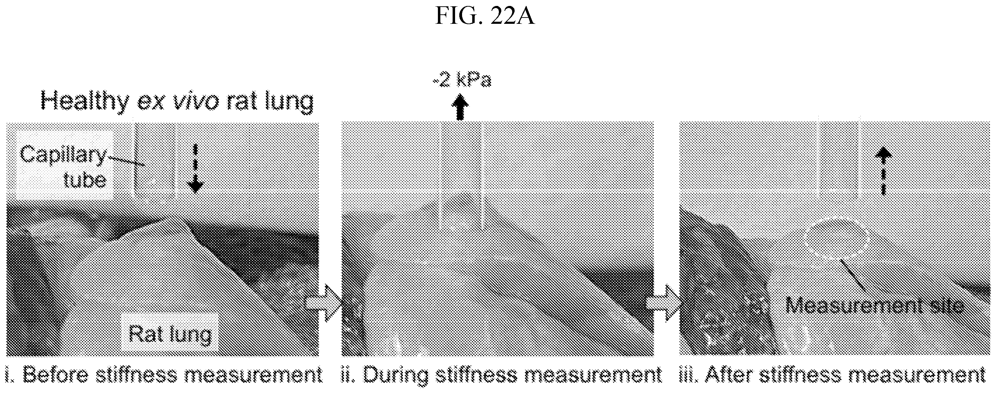

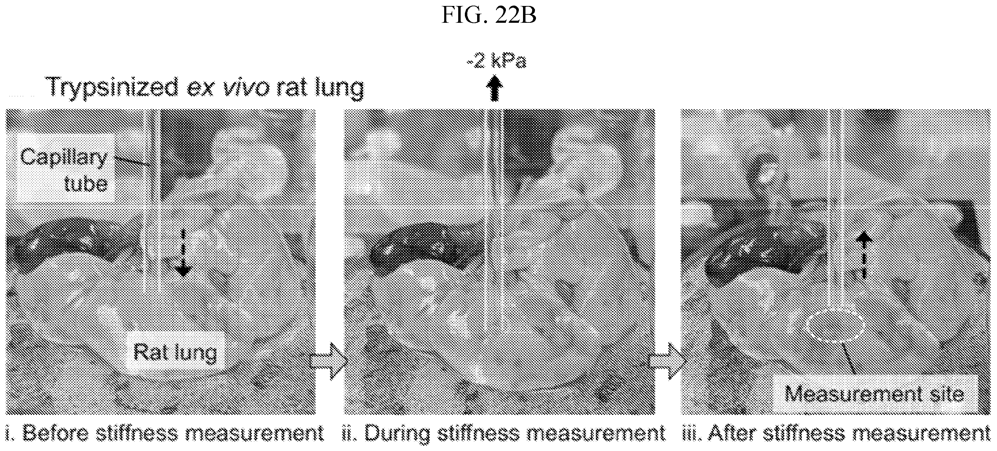

It was further investigated whether changes in the stiffness of lung parenchymal tissue caused by enzymatic disruption can be detected using the approach of the present invention. To induce acute tissue disruption, the lung was exposed to an enzymatic solution (i.e., trypsin) that can dislodge epithelial cells from ECM and further disrupt the surface tension in the alveolar space (See FIG. 11). The goal of trypsinization was not to mimic stereotypical presentation of any one pathology, but rather to assess the ability of the vacuum-assisted method to quantify changes in lung tissue stiffness. The rat lung was instilled with 0.25% trypsin (1 mL) with ICG dye through the trachea and incubated for 10 min. Distribution of the trypsin solution within the respiratory tract of the lung was confirmed through visualization of trypsin/ICG via NIR imaging of the lung. Static compliance (*C*_{S}) of the whole lung was monitored before and after the trypsin challenge by measuring its pressure-volume relation, where air pressure (*P*_{Alv}) and lung volume (*V*) were measured using a custom-built sensor module. Static compliance (*C*_{S}) was calculated by *C*_{S} = TV/(*P*_{Plat} - PEEP), where TV is tidal volume, *P*_{Plat} is plateau pressure, and PEEP is positive end-expiratory pressure. While the healthy lungs (control) displayed high static compliance (0.70 mL/cmH₂O), damaged lungs showed substantially reduced compliance (0.29 mL/cmH₂O), as trypsinized lungs required significantly greater pressures when ventilated with the same air volume (FIG. 11C). Histological analysis via H&E staining of the trypsinized lung showed excessive accumulation of fluid and debris in alveolar spaces with substantial reduction of intact cells and decreased number of nuclei compared to control lungs (FIG. 1D). In the trypsinized lungs, maximum elongation lengths under *P*v of -2 kPa were 0.11 ± 0.01 and 0.07 ± 0.01 mm, respectively, for 5 and 10 cmH₂O of *P*_{Alv} (FIG. 7A). As a result, elastic moduli of the enzymatically damaged lungs were determined to be 12.8 ± 1.0 and 21.7 ± 3.9 kPa, respectively, for 5 and 10 cmH₂O of *P*_{Alv}, indicating 73.1% and 64.6% increases in tissue stiffness compared to the control (healthy) lungs under the same measurement conditions. The significant increase in *E* following proteolytic tissue disruption, in particular for *P*_{Alv} of 5 cmH₂O (*p* = 0.003), suggests that the intrinsic deformation mechanics and stiffness of the lung tissue parenchyma was compromised following trypsin challenge. Further, in the damaged lungs, the effects of *P*_{Alv} on *E* were considerable (*p* = 0.02), highlighting the influence of measurement conditions such as intraalveolar pressure on tissue characterization. Notably, no physical damages to the lung parenchyma or pleura (e.g., blistering or rupturing) were observed in the visceral pleural layer of the lungs following the vacuum-based measurements (FIG. 22). FIG. 22 illustrates both healthy (FIG. 22A) and trypsinized rat lungs (FIG. 22B). Both were inspected visually for physical damage, such as blistering, that could occur due to negative pressure applied to the pleural surface. Photographs of lung taken during vacuum-based stiffness measurements (*P*_{V} = -2 kPa) showed that the pleural layer of the lungs remained intact with sign of physical damage created by the vacuum applied.

### Example 5: Compression-based Palpation Device

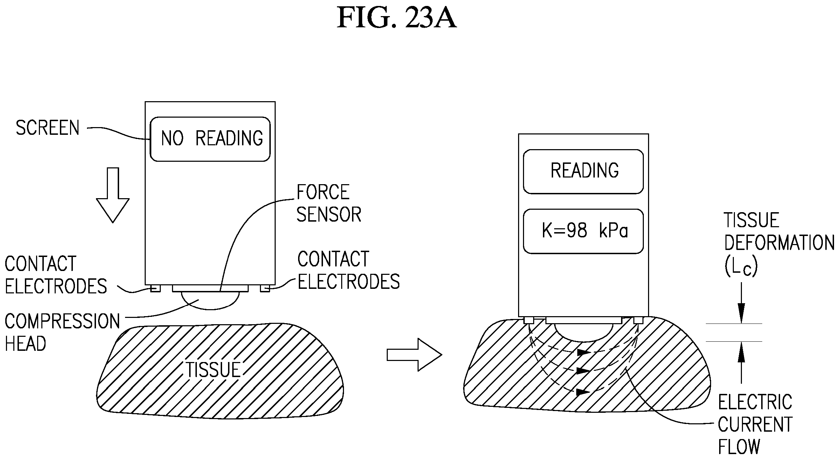

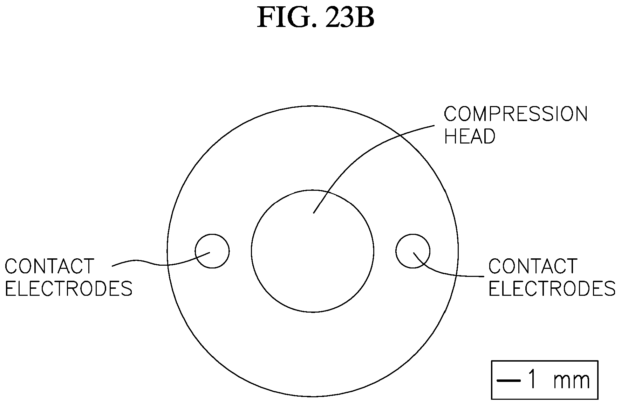

FIG. 23A is a schematic representation of compression-based tissue palpation device and its operation procedure. Contact electrodes ensure tight contact between the device tip and tissue during measurement as no electrical signal is detected if tissue compression is incomplete. A force sensor incorporated at the device tip measures the compression force (*F*) applied to the tissue. Compression head with a fixed and known height defines the tissue deformation length (*Lc*), allowing consistent tissue deformation across measurements. Tissue stiffness (*K*) is then calculated by relating *F* and *Lc.* FIG. 23B is a photograph showing the distal end of a prototype of a compression-based stiffness measurement device.

In an example, a device features a compression head with a fixed and known height that defines the tissue deformation length (*L*c), allowing consistent tissue deformation across measurements. The use of contact electrodes ensures tight contact between the device tip and tissue during measurement, as no electrical signal will be detected if tissue compression is incomplete. The force sensor incorporated at the device tip measures the compression force applied to the tissue. Such a device can be created into a portable, hand-held configuration for rapid assessments of external tissues or a steerable configuration for *in vivo* tissue assessments.

In such examples of the present invention, a measurement procedure can be followed: First, during tissue stiffness measurement, the device tip is gently pushed against the tissue. Next, tissue deformation continues until an electrical signal is detected via the contact electrodes. Finally, Tissue stiffness is calculated based on the tissue deformation (*L*c) and force (*F*) recorded at the time of electrical signal detection.

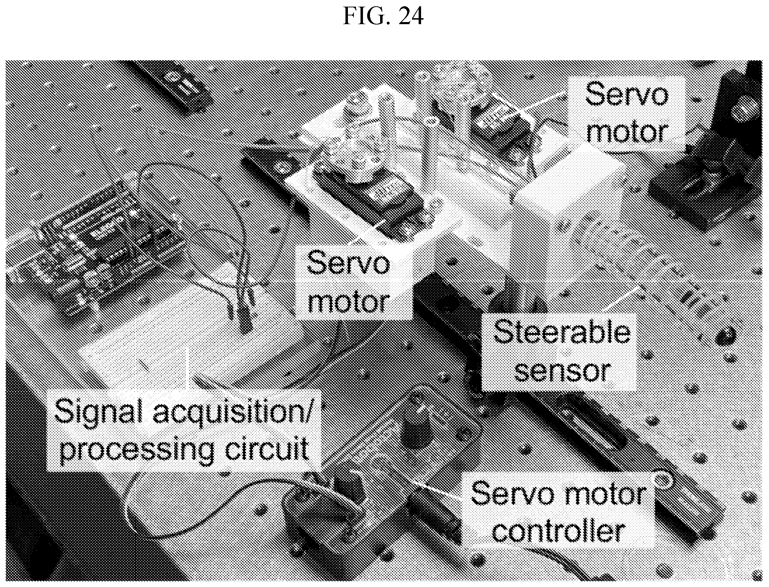

FIG. 24 is a photograph showing a prototype of a steerable compression-based tissue stiffness measurement device. The deflection and translational movement of the device is controlled by servo motors and a motor controller. Compression force measured via the force sensor and the electrical signal detected via the contact electrodes integrated at the distal end of the device is recorded and processed by an electric circuit.

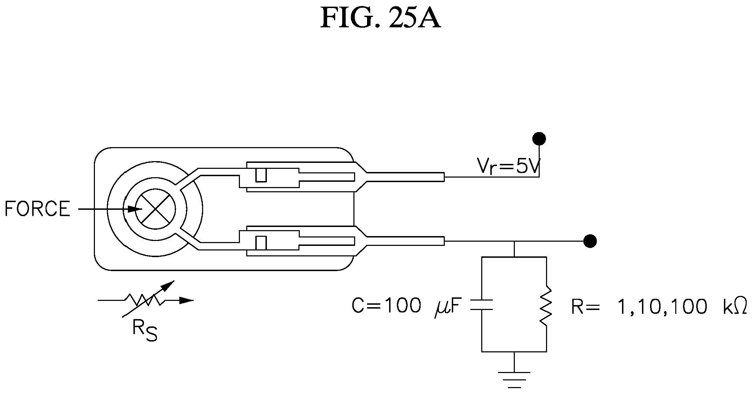

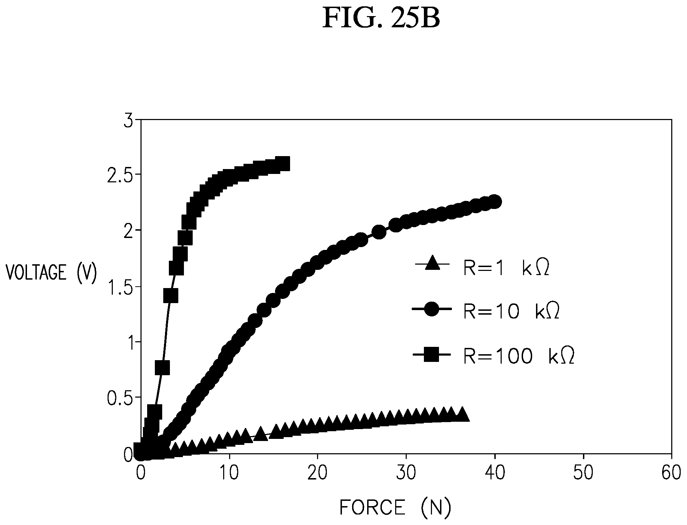

FIG. 25A is a schematic of the electrical circuit used to measure the force in the form of voltage (V). FIG. 25B is a series of calibration curves that correlate the measured voltage (*V*) and force (*F*) using different resistance values (*R*).

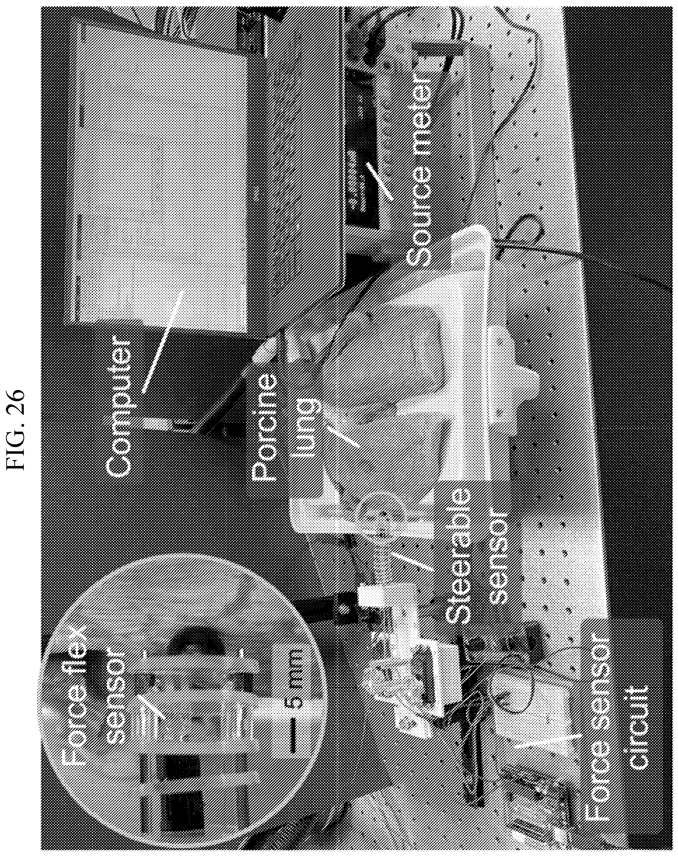

FIG. 26 is an experimental setup showing test of the compression-based tissue stiffness measurement device for measuring the stiffness of isolated porcine lung.

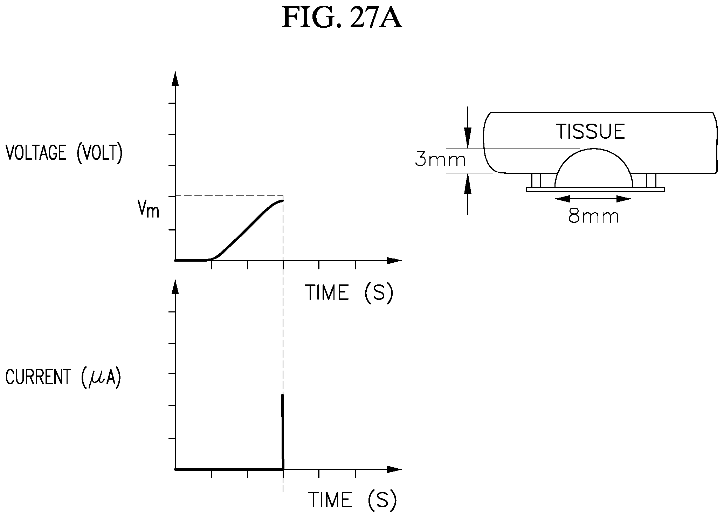

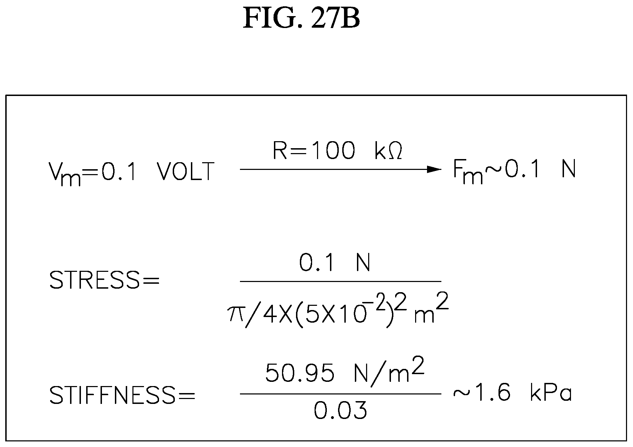

FIG. 27 shows stiffness measurement results of porcine lung using the compression-based device of the present invention. FIG. 27A is a schematic showing measurement of voltage and electrical current a via force sensor and contact electrodes, respectively, to determine the tissue stiffness. FIG. 27B is a table containing formulae for calculating tissue stiffness (i.e., modulus) using the measured electrical signals of FIG. 27A.

Additional details relating to the present invention are presented in the publication by Jiawen Chen et al. entitled "Non-destructive vacuum-assisted measurement of lung elastic modulus," Acta Biomater. 2021, Sep 1;131:370-380.

## Claims

1. A device for evaluating stiffness of materials, comprising:
a compression head;
an imaging element coupled to said compression head;
a motorized steering means adapted to move said imaging element and said compression head;
a pressure network adapted to apply positive or negative pressure to said compression head; and
a controller adapted to regulate and control said pressure network;
wherein the device further comprises an ablation means, **characterised in that** the ablation means includes a laser.

2. The device of Claim 1, wherein said pressure network comprises a pressure line, said pressure line and said imaging element being integrated with said motorized steering means as part of a steerable compartment of said device.

3. The device of Claim 1, wherein said imaging element comprises an optical fiber probe or a miniaturized camera.

4. The device of Claim 1, wherein said controller is adapted to regulate pressure applied to said compression head, analyze collected deformation data and calculate stiffness, or said controller utilizes computer vision.

5. The device of Claim 1, further comprising delivery means adapted to deliver fluorescent molecules.

6. The device of Claim 1, further comprising a balloon probe adapted to monitor pressure and volume.

7. The device of Claim 1, wherein said compression head is a dome-shaped tip, said compression head further including contact electrodes and a force sensor adapted to monitor compression force.

8. The device of Claim 1, wherein said controller is adapted to determine elongation length, deformation length or electrical resistance.

9. A method for stiffness measurement of a tissue of interest, comprising the steps of:
providing a probe, the probe having a compression head and an ablation means comprising a laser;
locating said probe such that said compression head is proximate the tissue of interest;
applying a pressure to said compression head;
detecting a response at the tissue of interest in response to said pressure applied via said applying step; and
calculating one or more physical properties of the tissue of interest based on said response.

10. The method of Claim 9, further comprising the step of regulating said pressure applied to said compression head or determining tissue stiffness via said calculating step.

11. The method of Claim 9, wherein the tissue of interest is a tumor, said method further comprising the steps of determining boundaries of said tumor in real-time and analyzing said tumor using computer vision.

12. The method of Claim 9, further comprising the steps of placing contact electrodes proximate the tissue of interest and measuring electrical resistance of the tissue of interest in response to said pressure.

13. The method of Claim 9, wherein said pressure is applied as suction force or compressive force, said method further comprising the step of measuring elongation length of the tissue of interest in response to said suction force, or the step of measuring tissue deformation length of the tissue of interest in response to said compressive force.

## Patentansprüche

1. Vorrichtung zur Bewertung von Steifigkeit von Materialien, umfassend:
einen Kompressionskopf;
ein Abbildungselement, das mit dem Kompressionskopf gekoppelt ist;
ein motorisiertes Lenkmittel, das ausgelegt ist, um das Abbildungselement und den Kompressionskopf zu bewegen;
ein Drucknetzwerk, das ausgelegt ist, um Über- oder Unterdruck auf den Kompressionskopf zu beaufschlagen; und
eine Steuerung, die ausgelegt ist, um das Drucknetzwerk zu regeln und zu steuern;
wobei die Vorrichtung ferner ein Ablationsmittel umfasst, **dadurch gekennzeichnet, dass** das Ablationsmittel einen Laser umfasst.

2. Vorrichtung nach Anspruch 1, wobei das Drucknetzwerk eine Druckleitung umfasst, wobei die Druckleitung und das Abbildungselement mit dem motorisierten Lenkmittel als Teil eines lenkbaren Einsatzes der Vorrichtung integriert sind.

3. Vorrichtung nach Anspruch 1, wobei das Abbildungselement eine optische Fasersonde oder eine Miniatur-Kamera umfasst.

4. Vorrichtung nach Anspruch 1, wobei die Steuerung ausgelegt ist, um Druck, der auf den Kompressionskopf beaufschlagt wird, zu regeln, gesammelte Verformungsdaten zu analysieren und die Steifigkeit zu berechnen, oder wobei die Steuerung Computervision verwendet.

5. Vorrichtung nach Anspruch 1, ferner umfassend ein Zufuhrmittel, das ausgelegt ist, um fluoreszierende Moleküle zuzuführen.

6. Vorrichtung nach Anspruch 1, ferner umfassend eine Ballonsonde, die ausgelegt ist, um den Druck und das Volumen zu überwachen.

7. Vorrichtung nach Anspruch 1, wobei der Kompressionskopf eine kuppelförmige Spitze ist, wobei der Kompressionskopf ferner Kontaktelektroden und einen Kraftsensor umfasst, der ausgelegt ist, um die Kompressionskraft zu überwachen.

8. Vorrichtung nach Anspruch 1, wobei die Steuerung ausgelegt ist, um die Dehnungslänge, die Verformungslänge oder den elektrischen Widerstand zu bestimmen.

9. Verfahren zur Steifigkeitsmessung eines Gewebes von Interesse, umfassend die folgenden Schritte:
Bereitstellen einer Sonde, wobei die Sonde einen Kompressionskopf und ein Ablationsmittel, umfassend einen Laser, umfasst;
Positionieren der Probe, sodass sich der Kompressionskopf in der Nähe des Gewebes von Interesse befindet;
Beaufschlagen eines Drucks auf den Kompressionskopf;
Detektieren einer Reaktion des Gewebes von Interesse als Reaktion auf den Druck, der über den Beaufschlagungsschritt beaufschlagt wird; und
Berechnen einer oder mehrerer physikalischer Eigenschaften des Gewebes von Interesse basierend auf der Reaktion.

10. Verfahren nach Anspruch 9, ferner umfassend den Schritt des Regelns des Drucks, der auf den Kompressionskopf beaufschlagt wird, oder des Bestimmens der Gewebesteifigkeit über den Berechnungsschritt.

11. Verfahren nach Anspruch 9, wobei das Gewebe von Interesse ein Tumor ist, wobei das Verfahren ferner die Schritte des Bestimmens von Grenzen des Tumors in Echtzeit und Analysieren des Tumors unter Verwendung von Computervision umfasst.

12. Verfahren nach Anspruch 9, ferner umfassend die Schritte des Positionierens von Kontaktelektroden in der Nähe des Gewebes von Interesse und des Messens des elektrischen Widerstands des Gewebes von Interesse als Reaktion auf den Druck.

13. Verfahren nach Anspruch 9, wobei der Druck als Saugkraft oder Kompressionskraft beaufschlagt wird, wobei das Verfahren ferner den Schritt des Messens einer Dehnungslänge des Gewebes von Interesse als Reaktion auf die Saugkraft oder den Schritt des Messens der Gewebeverformungslänge des Gewebes von Interesse als Reaktion auf die Kompressionskraft umfasst.

## Revendications

1. Dispositif d'évaluation de la rigidité de matériaux, comprenant :
une tête de compression ;
un élément d'imagerie couplé à ladite tête de compression ;
un moyen de direction motorisé conçu pour déplacer ledit élément d'imagerie et ladite tête de compression ;
un réseau de pression conçu pour appliquer une pression positive ou négative à ladite tête de compression ; et
un dispositif de commande conçu pour réguler et commander ledit réseau de pression ; dans lequel le dispositif comprend en outre un moyen d'ablation **caractérisé en ce que** le moyen d'ablation comprend un laser.

2. Dispositif selon la revendication 1, dans lequel ledit réseau de pression comprend une ligne de pression, ladite ligne de pression et ledit élément d'imagerie étant intégrés auxdits moyens de direction motorisés en tant que partie d'un compartiment orientable dudit dispositif.

3. Dispositif selon la revendication 1, dans lequel ledit élément d'imagerie comprend une sonde à fibre optique ou une caméra miniaturisée.

4. Dispositif selon la revendication 1, dans lequel ledit dispositif de commande est conçu pour réguler la pression appliquée à ladite tête de compression, analyser des données de déformation collectées et calculer la rigidité, ou ledit dispositif de commande utilise la vision par ordinateur.

5. Dispositif selon la revendication 1, comprenant en outre des moyens d'administration conçus pour administrer des molécules fluorescentes.

6. Dispositif selon la revendication 1, comprenant en outre une sonde à ballonnet conçue pour surveiller la pression et le volume.

7. Dispositif selon la revendication 1, dans lequel ladite tête de compression est une pointe en forme de dôme, ladite tête de compression comprenant en outre des électrodes de contact et un capteur de force conçu pour surveiller la force de compression.

8. Dispositif selon la revendication 1, dans lequel ledit dispositif de commande est conçu pour déterminer la longueur d'allongement, la longueur de déformation ou la résistance électrique.

9. Procédé de mesure de rigidité d'un tissu d'intérêt, comprenant les étapes de :
fourniture d'une sonde, la sonde ayant une tête de compression et un moyen d'ablation comprenant un laser ;
localisation de ladite sonde de sorte que ladite tête de compression soit proche du tissu d'intérêt ;
application d'une pression à ladite tête de compression ;
détection d'une réponse au niveau du tissu d'intérêt en réponse à ladite pression appliquée via ladite étape d'application ; et
calcul d'une ou de plusieurs propriétés physiques du tissu d'intérêt sur la base de ladite réponse.

10. Procédé selon la revendication 9, comprenant en outre l'étape de régulation de ladite pression appliquée à ladite tête de compression ou de détermination de la rigidité tissulaire via ladite étape de calcul.

11. Procédé selon la revendication 9, dans lequel le tissu d'intérêt est une tumeur, ledit procédé comprenant en outre les étapes de détermination des limites de ladite tumeur en temps réel et d'analyse de ladite tumeur en utilisant la vision par ordinateur.

12. Procédé selon la revendication 9, comprenant en outre les étapes de placement d'électrodes de contact à proximité du tissu d'intérêt et de mesure de la résistance électrique du tissu d'intérêt en réponse à ladite pression.

13. Procédé selon la revendication 9, dans lequel ladite pression est appliquée en tant que force d'aspiration ou force de compression, ledit procédé comprenant en outre l'étape de mesure de la longueur d'allongement du tissu d'intérêt en réponse à ladite force d'aspiration, ou l'étape de mesure de la longueur de déformation de tissu du tissu d'intérêt en réponse à ladite force de compression.
